(19) Europäisches Patentamt
European Patent Office
Office européen des brevets

(11) Veröffentlichungsnummer: **0 655 440 A2**

# (12) EUROPÄISCHE PATENTANMELDUNG

(21) Anmeldenummer: **94115800.8**

(22) Anmeldetag: **07.10.94**

(51) Int. Cl.⁶: **C07D 209/36**, C07D 209/08, A61K 31/40

(30) Priorität: **22.10.93 CH 3201/93**

(43) Veröffentlichungstag der Anmeldung: **31.05.95 Patentblatt 95/22**

(84) Benannte Vertragsstaaten: **AT BE CH DE DK ES FR GB GR IE IT LI LU MC NL PT SE**

(71) Anmelder: **F. HOFFMANN-LA ROCHE AG**
**Postfach 3255**
**CH-4002 Basel (CH)**

(72) Erfinder: **Bös, Michael**
**8A Marktgasse**
**CH-4310 Rheinfelden (CH)**

(74) Vertreter: **Poppe, Regina et al**
**Postfach 3255**
**CH-4002 Basel (CH)**

## (54) Indol derivate.

(57) Die Erfindung betrifft Verbindungen der allgemeinen Formel

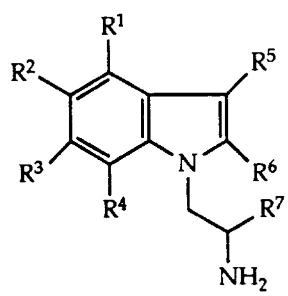

I

worin $R^1$ bis $R^4$ Wasserstoff, Halogen, nieder-Alkyl, Cycloalkyl oder Trifluormethyl, $R^5$ und $R^6$ Wasserstoff, Halogen, nieder-Alkyl, Cycloalkyl, Trifluormethyl, Hydroxy oder nieder-Alkoxy und $R^7$ Wasserstoff oder nieder-Alkyl bedeuten,
sowie pharmazeutisch annehmbare Säureadditionsalze der Verbindungen der Formel I.

Diese Verbindungen eignen sich insbesondere zur Behandlung oder Verhütung von zentralnervösen Störungen wie Depressionen, bipolaren Störungen, Angstzuständen, Schlaf- und Sexualstörungen, Psychosen, Schizophrenie, Migräne und andere mit Kopfschmerz oder Schmerz anderer Art verbundene Zustände, Persönlichkeitsstörungen oder obsessiv-compulsive Störungen, soziale Phobien oder panische Anfälle, mentale organische Störungen, mentale Störungen in Kindesalter, Aggressivität, alterbedingte Gedächnisstörungen und Verhaltensstörungen, Sucht, Obesitas, Bulimie etc., Schädigungen des Nervensystems durch Trauma, Schlaganfall, neurodegenerative Erkrankungen etc; cardiovasculären Störungen, wie Bluthochdruck, Trombose, Schlaganfall, und gastrointestinalen Störungen, wie Dysfunktion der Motilität des Gastrointestinaltrakts.

EP 0 655 440 A2

Die vorliegende Erfindung betrifft Indol-Derivate. Im speziellen betrifft sie Indol-Derivate der allgemeinen Formel

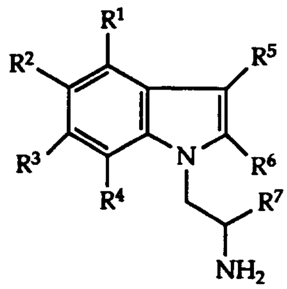

I

worin $R^1$ bis $R^4$ Wasserstoff, Halogen, nieder-Alkyl, Cycloalkyl oder Trifluormethyl, $R^5$ und $R^6$ Wasserstoff, Halogen, nieder-Alkyl, Cycloalkyl, Trifluormethyl, Hydroxy oder nieder-Alkoxy und $R^7$ Wasserstoff oder nieder-Alkyl bedeuten,
sowie pharmazeutisch annehmbare Säureadditionsalze der Verbindungen der Formel I.

Die Verbindungen und Salze sind neu und zeichnen sich durch wertvolle therapeutische Eigenschalten aus.

Sie eignen sich insbesondere zur Behandlung oder Verhütung von zentralnervösen Störungen wie Depressionen, bipolaren Störungen, Angstzuständen, Schlaf- und Sexualstörungen, Psychosen, Schizophrenie, Migräne und andere mit Kopfschmerz oder Schmerz anderer Art verbundene Zustände, Persönlichkeitsstörungen oder obsessiv-compulsive Störungen, soziale Phobien oder panische Anfälle, mentale organische Störungen, mentale Störungen in Kindesalter, Aggressivität, altersbedingte Gedächnisstörungen und Verhaltensstörungen, Sucht, Obesitas, Bulimie etc., Schädigungen des Nervensystems durch Trauma, Schlaganfall, neurodegenerative Erkrankungen etc; cardiovasculären Störungen, wie Bluthochdruck, Thrombose, Schlaganfall, und gastrointestinalen Störungen, wie Dysfunktion der Motilität des Gastrointestinaltrakts.

Gegenstand der vorliegenden Erfindung sind Verbindungen der Formel I und pharmazeutisch annehmbare Säureadditionssalze davon als solche und als pharmazeutische Wirkstoffe, die Herstellung dieser Verbindungen und Salze, ferner Arzneimittel, enthaltend eine Verbindung der Formel I oder ein pharmazeutisch annehmbares Säureadditionssalz davon, die Herstellung solcher Arzneimittel und die Verwendung der Verbindungen der Formel I und ihrer pharmazeutisch annehmbaren Salze bei der Bekämpfung oder Verhütung von Krankheiten, insbesondere von Krankheiten und Störungen der eingangs erwähnten Art, bzw. zur Herstellung entsprechender Arzneimittel.

Weiterhin sind Gegenstand der Erfindung Verbindungen der allgemeinen Formel

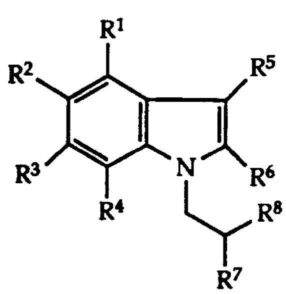

II

worin $R^1$ bis $R^7$ die obengenannten Bedeutungen haben und $R^8$ einen in eine Aminogruppe überführbaren Rest, eine Abgangsgruppe oder eine Hyroxygruppe bedeutet.
Diese Verbindungen sind wichtige Zwischenprodukte zur Herstellung der pharmazeutisch wertvollen Verbindungen der allgemeinen Formel I.

Der Ausdruck "nieder" bezeichnet Reste mit höchstens 7, vorzugsweise bis 4 Kohlenstoffatomen, "Alkyl" bezeichnet gradkettige oder verzweigte, gesättigte Kohlenwasserstoffreste wie Methyl, Ethyl, Isopropyl oder t-Butyl und Alkoxy bezeichnet eine über ein Sauerstoffatom gebundene Alkylgruppe wie Methoxy, Ethoxy, Propoxy, Isopropoxy oder Butoxy. "Halogen" kann Cl, Br, F oder J bedeuten.

Der Ausdruck "pharmazeutisch annehmbare Säureadditionssalze" umfasst Salze mit anorganischen und organischen Säuren, wie Salzsäure, Bromwasserstoffsäure, Salpetersäure, Schwefelsäure, Phosphorsäure, Zitronensäure, Ameisensäure, Fumarsäure, Maleinsäure, Essigsäure, Bernsteinsäure, Weinsäure, Methansulfonsäure, p-Toluolsulfonsäure und dergleichen.

$R^7$ kann zweckmässigerweise nieder-Alkyl, vorzugsweise Methyl bedeuten. Ebenso bevorzugt sind Verbindungen , worin $R^7$ Wasserstoff bedeutet. Bedeutet $R^7$ Methyl, sind Verbindungen besonders bevorzugt, worin $R^5$ und $R^6$ Wasserstoff bedeuten.

Weiterhin sind Verbindungen bevorzugt, worin $R^1$ Wasserstoff oder Methyl, $R^2$ Wasserstoff oder Fluor, $R^3$ Wasserstoff oder Chlor und $R^4$ Wasserstoff bedeuten.

Einige im Rahmen der vorliegenden Erfindung ganz besonders bevorzugte Vertreter der durch die allgemeine Formel I definierten Stoffklasse sind:

2-(5-Fluorindol-1-yl)-ethylamin
2-(6-Chlor-5-fluor-indol-1-yl)-ethylamin
2-(4-Methyl-3-methoxy-indol-1-yl)-ethylamin
(S)-2-(6-Chlor-5-fluor-indol-1-yl)-1-methyl-ethylamin

Beispiele weiterer bevorzugter Verbindungen der allgemeinen Formel I sind:

2-(4-Chlor-5-fluor-3-methoxy-indol-1-yl)-ethylamin,
2-(5-fluor-3-methoxy-indol-1-yl)-ethylamin,
2-(5-Chlor-indol-1-yl)-ethylamin,
2-(4-Chlor-5-fluor-indol-1-yl)-ethylamin,
(RS)-2-(5-Chlor-indol-1-yl)-1-methyl-ethylamin,
(RS)-2-(5-Fluor-indol-1-yl)-1-methyl-ethylamin,
(RS)-2-(6-Chlor-5-fluor-indol-1-yl)-1-methyl-ethylamin,
(R)-2-(5-Fluor--indol-1-yl)-1-methyl-ethylamin,
(S)-2-(6-Chlor-5-fluor-indol-1-yl)-1-methyl-ethylamin,
(R)-2-(6-Chlor-5-fluor-indol-1-yl)-1-methyl-ethylamin,
(RS)-2-(4-Methyl-indol-1-yl)-1-methyl-ethylamin,
(RS)-2-(5-Brom-indol-1-yl)-1-methyl-ethylamin,
(RS)-2-(6-Fluor-indol-1-yl)-1-methyl-ethylamin,
(S)-2-(5,6-Difluor-indol-1-yl)-1-methyl-ethylamin,
(R)-2-(5,6-Difluor-indol-1-yl)-1-methyl-ethylamin,
(S)-2-(5-Fluor-4-trifluormethyl-indol-1-yl)-1-methyl-ethylamin,
(S)-2-(5-Fluor-6-trifluormethyl-indol-1-yl)-1-methyl-ethylamin,
(S)-2-(4-Chlor-5-fluor-indol-1-yl)-1-methyl-ethylamin,
(R)-2-(4-Chlor-5-fluor-indol-1-yl)-1-methyl-ethylamin,

Verbindungen der allgemeinen Formel I sowie ihre pharmazeutisch annehmbaren Säureadditionssalze können erfindungsgemäss hergestellt werden, indem man

a) eine Verbindung der allgemeinen Formel

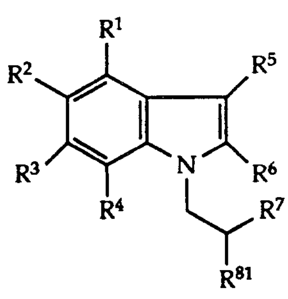

IIa

worin $R^1$ bis $R^7$ die oben angegebenen Bedeutungen haben und $R^{81}$ einen in eine Aminogruppe überführbaren Rest bedeutet, in die entsprechende Aminoverbindung überführt, oder,

b) eine Verbindung der allgemeinen Formel

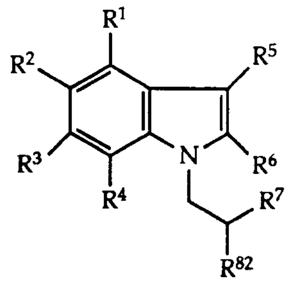

IIb

worin $R^1$ bis $R^7$ die oben angegebenen Bedeutungen haben und $R^{82}$ eine Abgangsgruppe bedeutet, mit Ammoniak umsetzt; und

c) erwünschtenfalls die erhaltene Verbindung der Formel I in pharmazeutisch annehmbare Säureadditionssalze überführt.

Die Verbindungen der allgemeine Formel IIa, worin $R^{81}$ einen in eine Aminogruppe überführbaren Rest, vorzugsweise eine Azidgruppe bedeutet, aber auch eine Nitrogruppe sein kann, können nach an sich bekannten Methoden hergestellt werden.

Bedeutet $R^{81}$ eine Azidgruppe, erfolgt die Herstellung der Verbindungen der Formel I durch Reduktion. Diese Reduktion kann mit komplexen Hydriden, wie z.B. Lithiumaluminiumhydrid oder durch katalytische Hydrierung an Metallkatalysatoren, wie z.B. Platin oder Palladium, durchgeführt werden. Verwendet man Lithiumaluminiumhydrid als Reduktionsmittel, sind vor allem wasserfreier Aether oder Tetrahydrofuran als Lösungsmittel geeignet. Zweckmässigerweise kann man wie folgt vergehen: Nach dem Zutropfen der Verbindung IIa mit $R^{81}$ = $N_3$ zu einer Lösung, bestehend aus dem wasserfreien Lösungsmittel und dem Hydrid, wird am Rückfluss gekocht, anschliessend mit wässriger Aether- oder THF-Lösung hydrolysiert und der Aluminium- und Lithiumhydroxid-Niederschlag mit THF ausgekocht.

Die katalytische Hydrierung an Metallkatalysatoren, z.B. Platin oder Palladium, erfolgt bei Zimmertemperatur. Als Lösungsmittel sind dabei besonders geeignet: Wasser, Alkohole, Essigester, Dioxan, Eisessig oder Gemische dieser Lösungsmittel.

Die Hydrierung erfolgt unter Wasserstoffatmosphäre entweder in einem Autoklaven oder in einer Schüttelapparatur.

Verbindungen der allgemeinen Formel I können auch hergestellt werden, wenn Verbindungen der Formel IIb, wobei $R^{82}$ eine Abgangsgruppe, z.B. Halogen, insbesondere Brom, bedeutet, mit Ammoniak umgesetzt werden.

Dazu werden die Verbindungen der Formel IIb zweckmässigerweise in flüssigem Ammoniak suspendiert und unter Erwärmung im Autoklaven gerührt. Nach dem Abdampfen des Ammoniaks wird der Rückstand in einem Lösungsmittel, vorzugsweise Dichlormethan, aufgenommen, gewaschen und getrocknet.

Es hat sich gezeigt, dass für eine pharmazeutische Verwendung dieser Verbindungen ihre Säureadditionssalze besonders gut geeignet sind. Besonders geeignet sind Verbindungen der Formel I in Form der Salze der Fumarsäure, aber auch alle anderen in der Beschreibung erwähnten Säuren bilden pharmazeutisch annehmbare Säureadditionssalze.

Die Zugabe der entsprechenden Säuren zu den Verbindungen der Formel I erfolgt zweckmässigerweise vor ihrer endgültigen Isolierung im Anschluss an die beschriebenen Herstellungsvarianten.

Die als Ausgangstoffe für die Herstellung der Verbindungen der allgemeine Formel IIa und IIb verwendeten Indolderivate können beispielsweise gemäss den nachfolgenden Reaktionschemata nach an sich bekannten Methoden hergestellt werden, z.B. nach der Fischer-Indol-Synthese, wo Arylhydrazone von Aldehyden oder Ketonen unter Einfluss von Säuren oder Metallhalogeniden als Katalysator unter Abspaltung von Ammoniak cyclisieren:

Schema 1

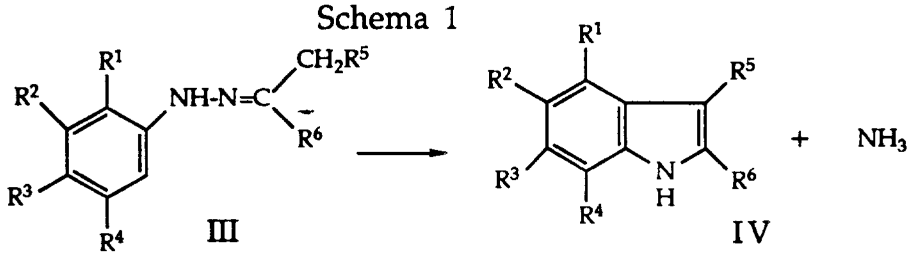

III IV

Dabei haben die Substituenten $R^1$ bis $R^6$ die obige Bedeutung.

In Analogie zu Synthesis, 1985, S. 186 können die entsprechenden Indolderivate auch nach folgendem Schema hergestellt werden:

Schema 2

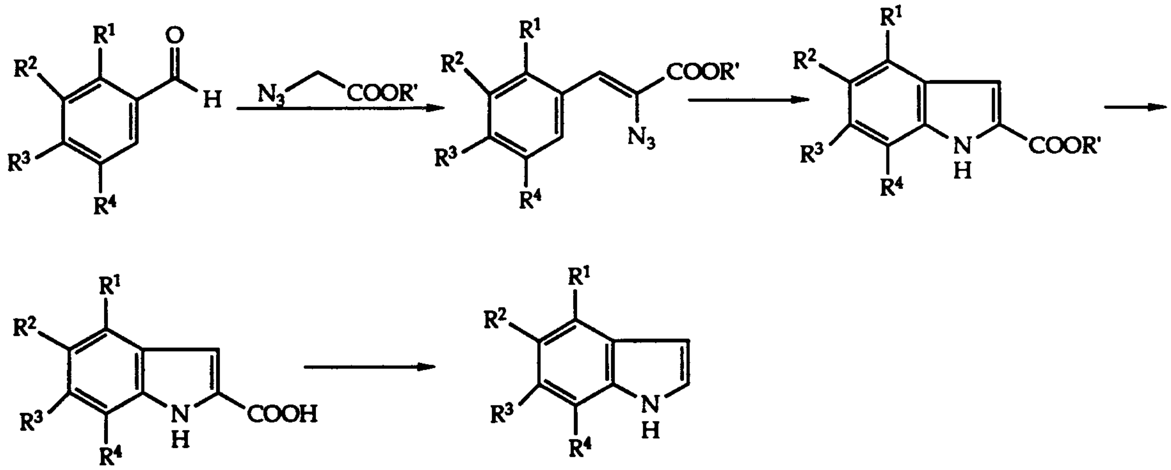

Dabei haben $R^1$ bis $R^4$ die oben genannten Bedeutungen und R' ist niederAlkyl.

Aromatische Aldehyde werden durch eine Alkoxid-katalysierte Kondensation mit Azidoessigsäure-Alkylestern in einstufiger Reaktion und guten Ausbeuten zu $\alpha$-Azidozimtsäure-Alkylestern umgesetzt.

Eine Thermolyse in siedendem p-Xylol führt anschliessend in fast quantitativer Ausbeute zu den Indol-2-carbonsäure-alkylestern. Die Ester-Gruppe kann nach an sich bekannten Methoden hydrolisiert und die Säuregruppe thermisch abgespalten werden.

Weiterhin kann man zu den Indolverbindungen gelangen, wenn entsprechend substituiertes Phenylglycin cyclisiert wird, wie das folgende Schema 3 zeigt:

Schema 3

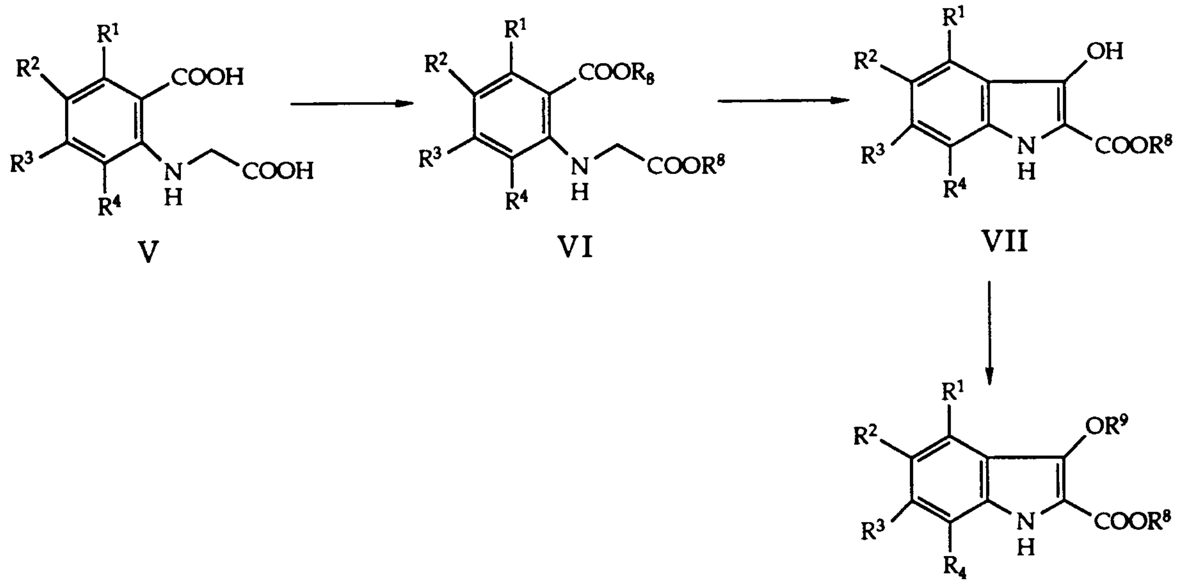

V VI VII

VIII

$R^1$ bis $R^4$ haben die oben genannten Bedeutungen und $R^8$ und $R^9$ bedeuten niederes Alkyl, wobei $R^8$ und $R^9$ gleich oder verschieden sein können. Die Verbindungen der Formel V können mit Alkoholen nach an sich bekannten Methoden in die Verbindungen der Formel VI umgewandelt werden. Durch Cyclisierung einer Verbindung der Formel VI erhält man ein Indol der Formel VII (J. Het. Chem. 16, 221 (1979)). Durch Umsetzung eines Indols der Formel VII mit einem Alkylierungsmittel, beispielsweise mit einem Dialkylsulfat oder mit Diazomethan, erhält man die Verbindungen der Formel VIII. Diese Umsetzung erfolgt in alkoholischen Lösungsmitteln, vorzugsweise Methanol, bei Raumtemperatur.

Eine weitere Herstellungsmöglichkeit für entsprechende Indole als Ausgangstoffe für die Herstellung der Verbindungen der allgemeinen Formel I zeigt der Schema 4.

Auch hier wird vom entsprechend substituierten Phenylglycin der Formel V ausgegangen.

Schema 4

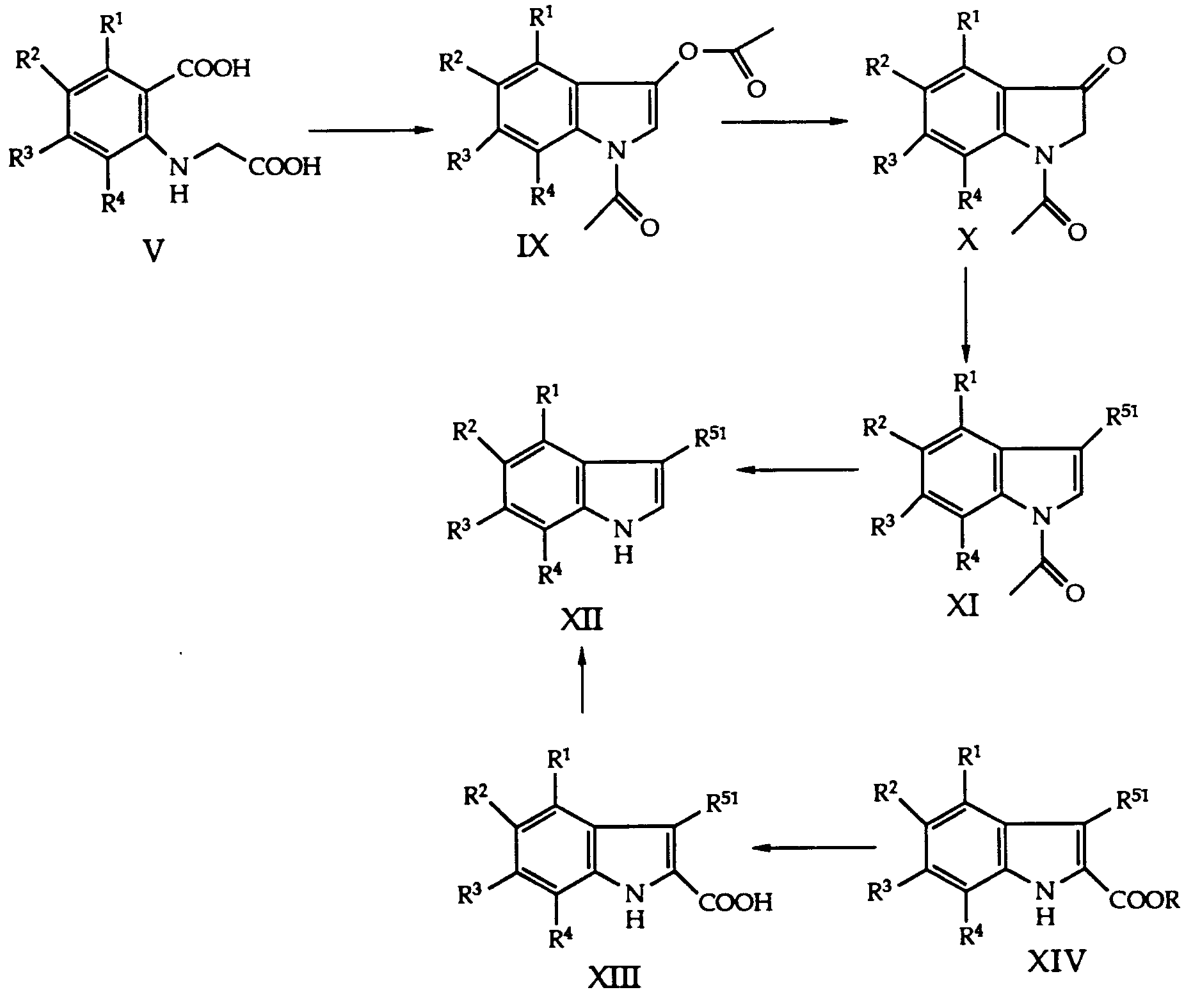

V IX X XI XII XIII XIV

Die Substituentenbezeichnungen für $R^1$ bis $R^4$ entsprechen den oben genannten, $R^{51}$ bedeutet nieder-Alkoxy und R bedeutet nieder-Alkyl.

Eine Verbindung der Formel V kann mit einem Acetat, vorzugsweise Natriumacetat, in Essigsäureanhydrid unter Rückfluss in eine Verbindung der Formel IX überführt werden, wobei diese anschliessend durch Hydrolyse, beispielsweise mit konzentrierter Schwefelsäure, in eine Verbindung der Formel X überführt werden kann. Die Verbindungen der Formel X sind bekannt (siehe Proc. Roy. Soc. London, 178 B, 781 (1958)) oder können auf analoge Weise hergestellt werden.

Eine Alkylierung einer Verbindung der Formel X kann mit üblichen Alkylierungsmitteln, wie z.B. Dialkylsulfaten, erfolgen. Eine Methylierung erfolgt vorzugsweise mit Dimethylsulfat zu einer Verbindung der Formel XI analog der im Bull. Soc. Chem. France 1978, 651 erschienenen Veröffentlichung.

Die Abspaltung der N-Acetylgruppe kann mit üblichen Methoden erfolgen, beispielsweise durch Reaktion und Natriumalkoholat in einem alkoholischen Lösungsmittel, vorzugsweise Natriummethylat in Methanol.

Eine weitere Möglichkeit der Herstellung der Verbindungen der Formel XII besteht in der Hydrolyse der Indolester der allgemeinen Formel XIV zu den Säuren der Formel XIII. Zweckmässigerweise verwendet man dabei Alkalihydroxid.

Die Decarboxylierung der Verbindungen der Formel XIII zu den Verbindungen der Formel XII kann durch Einwirkung von Temperaturen zwischen 300 bis 320 °C in einem Metallbad erfolgen.

Ausgehend von den beschriebenen Indolverbindungen der allgemeinen Formel IV erhält man nach Schema 5 Verbindungen der Formel II.

Schema 5

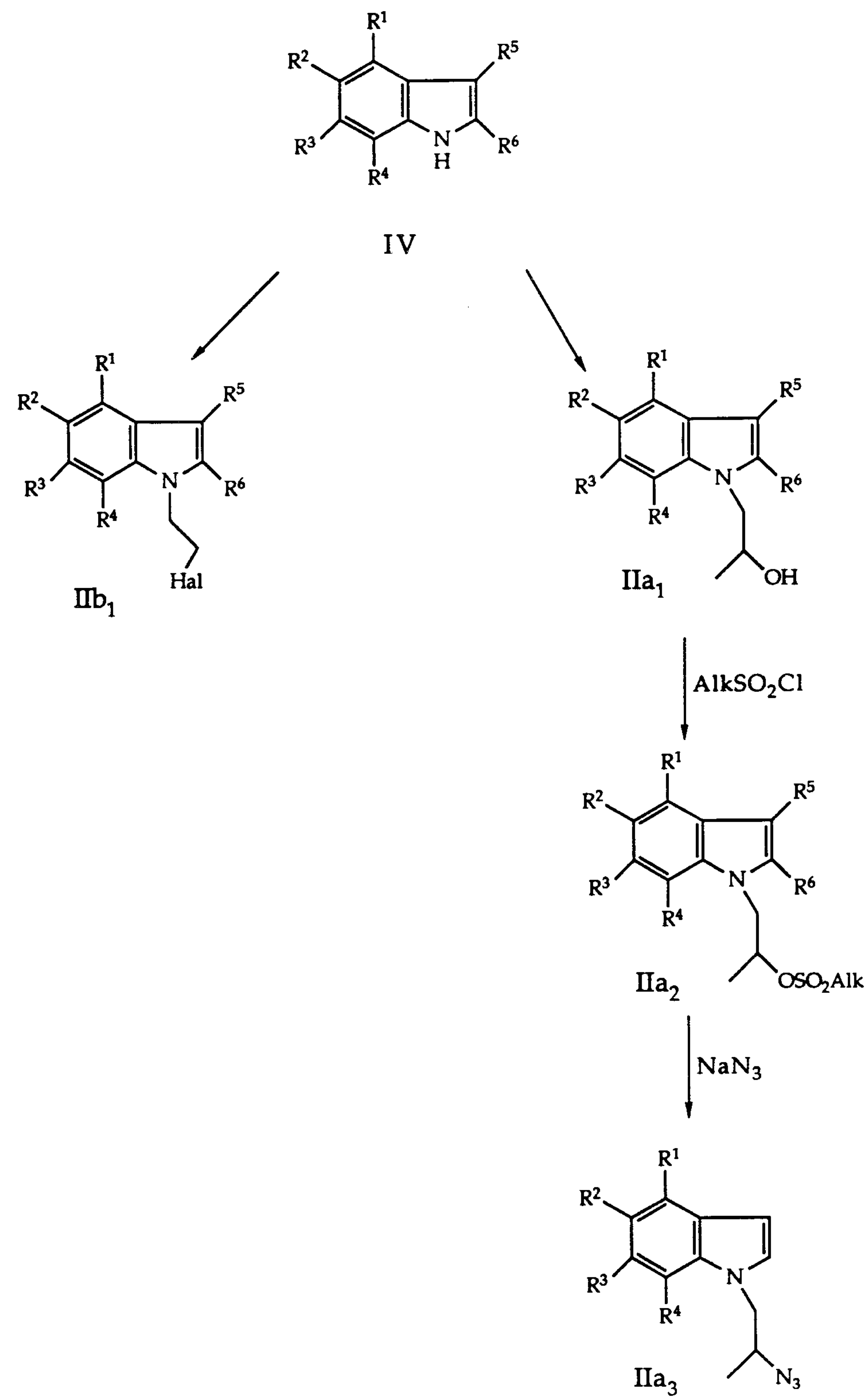


IV

$IIb_1$

$IIa_1$

$IIa_2$

$IIa_3$

Hierbei haben die Substituenten $R^1$ bis $R^6$ die oben angegebenen Bedeutungen.

Durch Behandeln einer Verbindung der Formel IV mit einem geeigneten Alkylierungsmittel, vorzugsweise 1,2-Dibromethan, erhält man eine Verbindung der Formel $IIb_1$. Diese Umsetzung wird zweckmässigerweise unter Bedingungen der Phasentransferkatalyse durchgeführt: Die Umsetzung erfolgt unter Rühren in einem zwei-Phasensystem aus Wasser und einem mit Wasser nicht mischbaren organischen Lösungsmittel in Gegenwart einer starken Base und eines Phasentransferkatalysators. Zweckmässigerweise kann als

organisches Lösungsmittel 1,2-Dibromethan, wenn es gleichzeitig als Reagenz dient, eingesetzt werden. Als starke Base eignet sich zum Beispiel Kaliumhydroxid oder Natriumhydroxid. Es können die üblichen Phasentransferkatalysatoren eingesetzt werden. Geeignete Katalysatoren sind z.B. Benzyltrimethylammoniumchlorid, Tetrabutylammoniumbromid und ähnliche Verbindungen. Die Reaktion wird vorzugsweise in einem Temperaturbereich von ca 20°-80°C durchgeführt.

Die Verbindung der Formel $IIa_1$ kann z.B. durch Umsetzung von Verbindungen der Formel IV mit einem Epoxid hergestellt werden.

Die Verbindungen der Formel IV können vorzugsweise in einer Suspension, bestehend aus Natriumhydrid und Tetrahydrofuran, bei ca 0°C gelöst und anschliessend mit Alkyloxiran versetzt werden, wobei Verbindungen der Formel $IIa_1$ entstehen.

Die Hydroxygruppe kann nach an sich bekannten Methoden durch eine Abgangsgruppe ersetzt werden, beispielsweise durch die Umsetzung mit einem Sulfonsäurechlorid, vorzugsweise mit Methansulfonsäurechlorid, zum Sulfonat. Durch Behandlung mit einem Azid, vorzugsweise Natriumazid, in einem polaren Lösungsmittel wie z.B. DMF, können Verbindungen der Formel $IIa_2$ oder $IIb_1$ in die entsprechenden Azidoverbindungen übergeführt werden.

Wie eingangs erwähnt, besitzen die Verbindungen der Formel I und ihre pharmazeutisch anwendbaren Säureadditionssalze wertvolle pharmakodynamische Eigenschaften. Sie sind in der Lage, an Serotonin-Rezeptoren zu binden und eignen sich daher zur Behandlung oder Verhütung von Krankheiten und Störungen der eingangs erwähnten Art bzw. zur Herstellung entsprechender Arzneimittel.

Die Bindung von erfindungsgemässen Verbindungen der Formel I an die Serotonin-Rezeptoren wurde durch Standardmethoden in vitro bestimmt. Die Präparate wurden gemäss den nachfolgend angegebenen Tests geprüft:

Methode 1:

a) Für die Bindung an den $5HT_{1A}$-Rezeptor gemäss den $^3H$-8-OH-DPAT-Bindungs-Test nach der Methode von S.J. Peroutka, Biol. Psychiatry 20, 971-979 (1985).

b) Für die Bindung an den $5HT_{2C}$-Rezeptor gemäss dem $^3H$-Mesulergin-Bindungs-Test nach der Methode von A.Pazos et al., Europ. J. Pharmacol. 106, 539-546 bzw. D.Hoyer, Receptor Research 8, 59-81 (1988).

c) Für die Bindung an den $5HT_{2A}$-Rezeptor gemäss den $^3H$-Ketanserin-Bindungs-Test nach der Methode von J.E.Leysen, Molecular Pharmacology 21, 301-304 (1981).

Es wurden die $IC_{50}$-Werte der zu prüfenden Substanzen bestimmt, d.h. diejenige Konzentration in nMol, durch welche 50% des am Rezeptor gebundenen Liganden verdrängt werden.

Die so ermittelte Aktivität einiger erfindungsgemässer Verbindungen sowie diejenige einiger Vergleichsverbindungen ist aus der nachfolgenden Tabelle ersichtlich:

| Substanz | Testmethode | | |
|---|---|---|---|
| | a | b | c |
| Buspiron | 19.50 | 3700.0 | 990.0 |
| NAN-190 | 0.56 | 1800.0 | 581.0 |
| 5HT | 1.50 | 9.5 | 1730.0 |
| Metergolin | 4.80 | 5.5 | 64.9 |
| mCPP | 227,0 | 53.0 | 319.0 |
| RU 24969 | 8.0 | 159.0 | 2500.0 |
| CP93129 | 1620.00 | 2780.0 | 29200.0 |
| Ritanserin | 5740.00 | 37.0 | 3.1 |
| Pirenperon | 2870.00 | 37.0 | 2.9 |

| Substanz | Beispiel | Testmethode | | |
|---|---|---|---|---|
| | | a | b | c |
| A | 1 | 1300 | 87 | 1580 |
| B | 2 | 3330 | 43 | 573 |
| C | 3 | 5070 | 533 | 6330 |
| D | 4 | 309 | 194 | 2720 |
| E | 5 | 3680 | 23,5 | 320 |
| F | 6 | 3470 | 32 | 1310 |
| G | 7 | 2370 | 64 | 850 |
| H | 8 | 5650 | 63 | 2490 |
| I | 9 | 7220 | 81 | 740 |
| J | 10 | 2420 | 13.5 | 922 |
| K | 11 | 5070 | 113 | 1340 |
| L | 12 | inact. | 75 | 1430 |
| M | 14 | 3080 | 9.5 | 860 |
| N | 13 | 1570 | 44.5 | 893 |
| O | 15 | 8650 | 77 | 1140 |
| P | 16 | 4060 | 168 | 4000 |
| U | 24 | 5590 | 25 | 403 |
| Q | 18 | 2430 | 58 | 2700 |
| R | 20 | inact. | 47 | 2120 |
| S | 19 | 5010 | 94 | 3260 |
| T | 23 | 9450 | 14 | 402 |
| V | 25 | 4740 | 629 | 5060 |

Dabei bedeuten:

A 2-(5-Fluor-indol-1-yl)-ethylamin fumarat
B 2-(4-Chlor-5-fluor-3-methoxy-indol-1-yl)-ethylamin fumarat
C 2-(5-Fluor-3-methoxy-indol-1-yl)-ethylamin fumarat
D 2-(5-Chlor-indol-1-yl)-ethylamin fumarat
E 2-(4-Chlor-5-fluor-indol-1-yl)-ethylamin fumarat
F 2-(6-Chlor-5-fluor-indol-1-yl)-ethylamin fumarat
G 2-(4-Methyl-3-methoxy-indol-1-yl)-ethylamin fumarat
H (RS)-2-(5-Chlor-indol-1-yl)-1-methyl-ethylamin fumarat
I (RS)-2-(5-Fluor-indol-1-yl)-1-methyl-ethylamin fumarat
J (RS)-2-(6-Chlor-5-fluor-indol-1-yl)-1-methyl-ethylamin fumarat
K (R)-2-(5-Fluor-indol-1-yl)-1-methyl-ethylamin fumarat
L (S)-2-(5-Fluor-indol-1-yl)-1-methyl-ethylamin fumarat
M (S)-2-(6-Chlor-5-fluor-indol-1-yl)-1-methyl-ethylamin fumarat
N (R)-2-(6-Chlor-5-fluor-indol-1-yl)-1-methyl-ethylamin fumarat
O (RS)-2-(4-Methyl-indol-1-yl)-1-methyl-ethylamin fumarat
P (RS)-2-(5-Brom-indol-1-yl)-1-methyl-ethylamin fumarat
Q (RS)-2-(6-Fluor-indol-1-yl)-1-methyl-ethylamin fumarat
R (S)-2-(5,6-Difluor-indol-1-yl)-1-methyl-ethylamin fumarat
S (R)-2-(5,6-Difluor-indol-1-yl)-1-methyl-ethylamin fumarat
T (S)-2-(4-Chlor-5-fluor-indol-1-yl)-1-methyl-ethylamin fumarat
U (R)-2-(4-Chlor-5-fluor-indol-1-yl)-1-methyl-ethylamin fumarat
V (RS)-2-(5-Methyl-indol-1-yl)-1-methyl-ethylamin fumarat

Methode II:

a) Zur Bestimmung der Affinität einer Verbindung zum $5HT_{1A}$-Rezeptor wurden Verdrängungsversuche mit [3H]-5-HT (1 nM) als Radioligand an rekombinierten human-$5HT_{1A}$ Rezeptoren expremiert in 3T3-Zellen von Mäusen durchgeführt. Es wurden Membranen verwendet, die von $2x10^5$ Zellen erhalten wurden, sowie verschiedene Konzentrationen der jeweiligen Testverbindung.

b) Für die Bindung an den $5HT_{2C}$-Rezepter gemäss dem [3H]-5-HT-Bindungs-Test nach der Methode von S.J. Peroutka et al., Brain Research 584, 191-196 (1992).

c) Für die Bindung an den $5HT_{2A}$-Rezeptor gemäss dem [3H]-DOB-Bindungs-Test nach der Methode von T. Branchek et. al., Molecular Pharmacology 38, 604-609 (1990).

Es sind die $p_{ki}$-Werte ($p_{ki}$ = $-\log_{10}$ Ki) der zu prüfenden Substanzen angegeben. Der ki-Wert ist durch folgende Formel definiert:

$$Ki = \frac{IC_{50}}{1 + \frac{[L]}{K_D}}$$

wobei die $IC_{50}$-Werte diejenigen Konzentrationen der zu prüfenden Verbindungen in nM sind, durch welche 50% des am Rezeptor gebundenen Liganden verdrängt werden. [L] ist die Konzentration des Liganden und der $K_D$-Wert die Dissoziationskonstanta des Liganden.

Die so ermittelte Aktivität einiger erfindungsgemässer Verbindungen ist aus nachfolgender Tabelle ersichtlich:

| Testmethode | | | |
|---|---|---|---|
| **Beispiel No.** | **a** | **b** | **c** |
| 30 | 5.00 | 8.40 | 6.73 |
| 31 | 5.50 | 7.91 | 6.61 |
| 32 | 6.16 | 8.21 | 6,59 |
| 33 | 5.00 | 8.46 | 6.91 |
| 34 | 5.00 | 8.81 | 7.49 |
| 35 | 5.00 | 8.28 | 6.86 |
| 36 | 5.30 | 8.52 | 7.12 |
| 37 | 4.98 | 8.57 | 8.50 |
| 38 | <5 | 7.70 | 6.80 |

Dabei bedeuten:

30 (S)-2-(3-Ethyl-5-fluorindol-1-yl)-1-methyl-ethylamin fumarat,
31 (S)-2-(4-Isopropyl-5-fluorindol-1-yl)-1-methyl-ethylamin fumarat,
32 (S)-2-(6-Isopropyl-5-fluorindol-1-yl)-1-methyl-ethylamin fumarat,
33 (S)-2-(6-Chlor-5-fluor-3-ethylindol-1-yl)-1-methyl-ethylamin fumarat,
34 (S)-2-(4-Chlor-5-fluor-3-ethylindol-1-yl)-1-methyl-ethylamin fumarat,
35 (S)-2-(5-fluor-3-methylindol-1-yl)-1-methyl-ethylamin fumarat,
36 (S)-2-(6-Chlor-5-fluor-3-methylindol-1-yl)-1-methyl-ethylamin fumarat,
37 (S)-2-(5-Fluor-3-methoxy-4-methylindol-1-yl)-1-methyl-ethylamin fumarat,
38 (S)-2-(3-Methoxy-4-methylindol-1-yl)-1-methyl-ethylamin fumarat,

Peniserektionen (Ratten)

Es wurde gezeigt, dass die Peniserektion abhängig ist von der Stimulierung des $5HT_{2C}$ Rezeptors (vergl. Berendsen & Broekkamp, Eur. J. Pharmacol. 135, 179 - 184 (1987).

Innerhalb 45 Minuten nach Verabreichen der Testsubstanz an die Tiere wurde die Anzahl der Peniserektionen bestimmt. Die $ED_{50}$ ist die Dosis, die 50% dieser Erektionen hervorruft.

| Beispiel No. | $ED_{50}$ (mg/kg, s. c.) |
|---|---|
| 1 | 0,49 |
| 2 | 0,23 |
| 3 | 2.70 |
| 4 | 3.30 |
| 5 | 0.27 |
| 6 | 0.30 |

Die Verbindungen der Formel I und die pharmazeutisch annehmbaren Säureadditionssalze der Verbindungen der Formel I können als Heilmittel, z.B. in Form von pharmazeutischen Präparaten, Verwendung finden. Die pharmazeutischen Präparate können oral, z.B. in Form von Tabletten, Lacktabletten, Dragées, Hart- und Weichgelatinekapseln, Lösungen, Emulsionen oder Suspension, verabreicht werden. Die Verabreichung kann aber auch rektal, z.B. in Form von Suppositorien, parenteral, z.B. in Form von Injektionslösungen, oder nasal erfolgen.

Zur Herstellung von pharmazeutischen Präparaten können die Verbindungen der Formel I und die pharmazeutisch annehmbaren Säureadditionssalze der Verbindungen der Formel I mit pharmazeutisch inerten, anorganischen oder organischen Trägern verarbeitet werden. Als solche Träger kann man für Tabletten, Lacktabletten, Dragées und Hartgelatinekapseln beispielsweise Lactose, Maisstärke oder Derivate davon, Talk, Stearinsäure oder deren Salze und dergleichen verwenden. Für Weichgelatinekapseln eignen sich als Träger beispielsweise pflanzliche Oele, Wachse, Fette, halbfeste und flüssige Polyole und dergleichen. Je nach Beschaffenheit des Wirkstoffes sind jedoch bei Weichgelatinekapseln überhaupt keine Träger erforderlich. Zur Herstellung von Lösungen und Sirupen eignen sich als Träger beispielsweise Wasser, Polyole, Glyzerin, pflanzliches Oel und dergleichen. Für Suppositorien eignen sich als Träger beispielsweise natürliche oder gehärtete Oele, Wachse, Fette, halbflüssige oder flüssige Polyole und dergleichen.

Die pharmazeutischen Präparate können daneben noch Konservierungsmittel, Lösungsvermittler, Stabilisierungsmittel, Netzmittel, Emulgiermittel, Süssmittel, Färbemittel, Aromatisierungsmittel, Salze zur Veränderung des osmotischen Druckes, Puffer, Ueberzugsmittel oder Antioxidantien enthalten. Sie können auch noch andere therapeutisch wertvolle Stoffe enthalten.

Arzneimittel, enthaltend eine Verbindung der Formel I oder ein pharmazeutisch annehmbares Säureadditionssalz davon und einen therapeutisch inerten Träger, sind ebenfalls Gegenstand der vorliegenden Erfindung, ebenso ein Verfahren zu deren Herstellung, das dadurch gekennzeichnet ist, dass man eine oder mehrere Verbindungen der Formel I und/oder pharmazeutisch annehmbare Säureadditionssalze wertvolle Stoffe zusammen mit einem oder mehreren therapeutisch inerten Trägern in eine galenische Darreichungsform bringt.

Erfindungsgemäss kann man Verbindungen der allgemeinen Formel I sowie deren pharmazeutisch annehmbare Säureadditionssalze bei der Behandlung oder Verhütung von zentralnervösen Störungen, wie Depressionen, bipolaren Störungen, Angstzuständen, Schlaf- und Sexualstörungen, Psychosen, Schizophrenie, Migräne und andere mit Kopfschmerz oder Schmerz anderer Art verbundene Zustände, Persönlichkeitsstörungen oder obsessiv-compulsive Störungen, soziale Phobien oder panische Anfalle, mentale organische Störungen, mentale Störungen im Kindesalter, Aggressivität, altersbedingte Gedächtnisstörungen und Verhaltensstörungen, Sucht, Obesitas, Bulimie etc.; Schädigungen des Nervensystems durch Trauma, Schlaganfall, neurodegenerative Erkrankungen etc.; cardiovasculären Störungen, wie Bluthochdruck, Thrombose, Schlaganfall etc.; und gastrointestinalen Störungen, wie Dysfunktion der Motilität des Gastrointestinaltrakts bzw. zur Herstellung entsprechender Arzneimittel verwenden. Die Dosierung kann innerhalb weiter Grenzen variieren und ist natürlich in jedem einzelnen Fall den individuellen Gegebenheiten anzupassen. Bei oraler Verabreichung liegt die Dosis in einem Bereich von etwa 0,01 mg pro Dosis bis etwa 500 mg pro Tag einer Verbindung der allgemeinen Formel I bzw. der entsprechenden Menge eines pharmazeutisch annehmbaren Säureadditionssalzes davon, wobei aber die obere Grenze auch überschritten werden kann, wenn sich dies als angezeigt erweisen sollte.

Die nachfolgenden Beispiele dienen der näheren Erläuterung der vorliegenden Erfindung. Sie sollen deren Umfang jedoch in keiner Weise beschränken. Sämtliche Temperaturen sind in Celsiusgraden angegeben.

Beispiel 1

Eine Lösung von 9.8 g (72.6 mmol) 5-Fluorindol in 360 ml 1,2-Dibromethan wurde mit 180 ml 28%iger NaOH und 0.59 g (1.82 mmol) Tetrabutylammoniumbromid versetzt. Das Gemisch wurde 42 Stunden bei 50° gerührt. Die Phasen wurden getrennt und die wässrige Phase wurde mit Toluol extrahiert. Die vereinigten organischen Phasen wurden mit Wasser gewaschen und über Natriumsulfat getrocknet. Das Lösungsmittel wurde abdestilliert und der Rückstand wurde in 1.9 l flüssigem Ammoniak suspendiert und 15 Stunden bei 80° im Autoklaven gerührt. Nach dem Abdampfen des Ammoniaks wurde der Rückstand in 500 ml Dichlormethan aufgenommen und mit 100 ml Wasser und 100 ml gesättigter Kochsalzlösung gewaschen. Die organische Phase wurde über Natriumsulfat getrocknet und das Lösungsmittel wurde abdestilliert. Der Rückstand wurde über 350 g Kieselgel mit Essigester-Methanol (5:1) chromatographiert. Man erhielt 8.6 g rötliches Öl, das in 970 ml Ether gelöst und mit Aktivkohle versetzt wurde. Nach Filtration wurde die Lösung mit 5.6 g (48.2 mmol) Fumarsäure versetzt und 2 Tage gerührt. Die Kristalle wurden abfiltriert und getrocknet. Man erhielt 11.1 g (39.5 %) 2-(5-Fluoro-indol-1-yl)-ethylamin fumarat (1:1.8) mit Smp. 173-175° (Zers.)

Beispiel 2

a) Eine Suspension von 2.63 g (10.6 mmol) N-[3-Chlor-2-(hydroxycarbonyl)-4-fluor-phenyl]glycin und 2.63 g (32.06 mmol) Natriumacetat in 25 ml Essigsäureanhydrid wurde 45 min unter Rückfluss gekocht. Das Lösungsmittel wurde im Vakuum entfernt und der Rückstand wurde mit 50 ml Wasser versetzt. Die Kristalle wurden abfiltriert, mit Wasser gewaschen und getrocknet. Man erhielt 2.7 g (94%) 3-Acetoxy-1-acetyl-chlor-5-fluorindol als gelbe Kristalle mit Smp. 168-169°.

b) Zu 20 ml 90%iger Schwefelsäure fügte man 2.65 g (9.8 mmol) 3-Acetoxy-1-acetyl-chlor-5-fluorindol und verdünnte das Reaktionsgemisch nach dreiviertelstündigem Rühren mit 100 mi Eiswasser. Der Niederschlag wurde abfiltriert, mit Wasser gewaschen und getrocknet. Man erhielt 2 g (92.5%) 1-Acetyl-4-chlor-5-fluorindolin-3-on als hellbraune Kristalle mit einem Smp. von 203-204°.

c) Eine Mischung von 2 g (8.78 mmol) 1-Acetyl-4-chlor-5-fluorindolin-3-on, 2 g pulverisiertes Natriumhydroxid und 30 ml Dimethylsulfat wurde 5 Stunden bei Raumtemperatur gerührt. Nach Zugabe von 250 ml gesättigter Natriumbicarbonatlösung wurde über Nacht gerührt und anschliessend wurde abfiltriert. Der Rückstand wurde in 150 ml Ether und 100 ml Essigester abfiltriert. Die Lösung wurde über Natriumsulfat getrocknet, filtriert und eingedampft. Man erhielt 2 g (95%) 1-Acetyl-4-chlor-5-fluor-3-methoxyindol als grünliche Kristalle mit Smp. 114-115°.

d) Eine Lösung von 2 g (8.28 mmol) 1-Acetyl-4-chlor-5-fluor-3-methoxyindol und 0.48 (8,8 mmol) Natriummethylat in 35 ml Methanol wurde eine Stunde bei Raumtemperatur gerührt. Nach dem Entfernen des Lösungsmittels wurde mit Wasser und Ethylacetat extrahiert, die organische Phase mit gesättigter Kochsalzlösung gewaschen und über Natriumsulfat getrocknet. Das Lösungsmittel wurde entfernt und der Rückstand wurde über 100 g Kieselgel mit Hexan-Essigester (3:1) chromatographiert. Man erhielt 0.88 g (53 %) 4-Chlor-5-fluor-3-methoxyindol.

e) Eine Lösung von 2 g (10 mmol) 4-Chlor-5-fluor-3-methoxyindol in 50 ml 1,2-Dibromethan wurde mit 50 ml 28%iger NaOH und 100 mg (0.3 mmol) Tetrabutyl-ammoniumbromid versetzt. Das Gemisch wurde 15 Stunden gerührt. Die Phasen wurden getrennt und die wässrige Phase wurde mit Toluol extrahiert. Die vereinigten organischen Phasen wurden mit Wasser gewaschen und über Natriumsulfat getrocknet. Das Lösungsmittel wurde abdestilliert und der Rückstand wurde in 200 ml flüssigem Ammoniak suspendiert und 18 Stunden bei 80° im Autoklaven gerührt. Nach dem Abdampfen des Ammoniaks wurde der Rückstand in 50 ml Dichlormethan aufgenommen und mit Wasser und gesättigter Kochsalzlösung gewaschen. Die organische Phase wurde über Natriumsulfat getrocknet und das Lösungsmittel wurde abdestilliert. Der Rückstand wurde in 200 ml Ether gelöst und mit 0.7 g (6 mmol) Fumarsäure versetzt und 2 Tage gerührt. Die Kristalle wurden isoliert und getrocknet. Man erhielt 2 g (55 %) 2-(4-Chlor-5-fluoro-3-methoxy-indol-1-yl)-ethylamin fumarat (1:1) als beige Kristalle mit Smp. 195-196°.

Beispiel 3

a) Zu 20 ml 90%iger Schwefelsäure fügte man 2.2 g (10 mmol) 3-Acetoxy-1-acetyl-5-fluorindol und verdünnte das Reaktionsgemisch nach dreiviertelstündigem Rühren mit 100 ml Eiswasser. Der Niederschlag wurde abfiltriert, mit Wasser gewaschen und getrocknet. Man erhielt 1.6 g (83 %) 1-Acetyl-5-fluorindolin-3-on als beige Kristalle mit einem Smp. von 143-144°.

b) Eine Mischung von 1.6 g (8.3 mmol) 1-Acetyl-5-fluorindolin-3-on, 1.9 g pulverisiertes Natriumhydroxid und 28 ml Dimethylsulfat wurde zwei Stunden bei Raumtemperatur gerührt. Nach Zugabe von 240 ml gesättigter Natriumbicarbonatlösung wurde über Nacht gerührt und anschliessend wurde abfiltriert. Der Rückstand wurde in 100 ml Ether und 100 ml Essigester aufgenommen. Die Lösung wurde über Natriumsulfat getrocknet, filtriert und eingedampft. Man erhielt 1.47 g (86 %) 1-Acetyl-5-fluor-3-methoxy-indol als grünliche Kristalle mit Smp. 124-125°.

c) Eine Lösung von 1.25 g (6.03 mmol) 1-Acetyl-5-fluor-3-methoxyindol und 0.35 (6.5 mmol) Natriummethylat in 23 ml Methanol wurde eine Stunde bei Raumtemperatur gerührt. Nach dem Entfernen des Lösungsmittels wurde mit Wasser und Ethylacetat extrahiert, die organische Phase mit gesättigter Kochsalzlösung gewaschen und über Natriumsulfat getrocknet. Das Lösungsmittel wurde entfernt und der Rückstand wurde im Kugelrohr bei einer Badtemperatur von 200° und einem Druck von 50 mbar destilliert. Man erhielt 0.76 g (76%) 5-Fluor-3-methoxyindol.

d) Eine Lösung von 0.76 g (4.6 mmol) 5-Fluor-3-methoxyindol in 25 ml 1,2-Dibromethan wurde mit 25 ml 28%iger NaOH und 40 mg (0.12 mmol) Tetrabutyl-ammoniumbromid versetzt. Das Gemisch wurde 15 Stunden bei 50° gerührt. Die Phasen wurden getrennt und die wässrige Phase wurde mit Toluol extrahiert. Die vereinigten organischen Phasen wurden mit Wasser gewaschen und über Natriumsulfat getrocknet. Das Lösungsmittel wurde abdestilliert und der Rückstand wurde über 60 g Aluminiumoxid mit Hexan-Essigester (5:1) chromatographiert. Man erhielt ein gelbes Öl, das in 80 ml flüssigem Ammoniak suspendiert und 18 Stunden bei 80° im Autoklaven gerührt wurde. Nach dem Abdampfen des Ammoniaks wurde der Rückstand in Dichlormethan aufgenommen und mit Wasser und gesättigter Kochsalzlösung gewaschen. Die organische Phase wurde über Natriumsulfat getrocknet und das Lösungsmittel wurde abdestilliert. Der Rückstand wurde in 90 ml Ether und 3 ml Methanol gelöst und mit 0.35 g (3 mmol) Fumarsäure versetzt und 2 Tage gerührt. Die Kristalle wurden abfiltriert und getrocknet. Man erhielt 0.56 g (37.5 %) 2-(5-fluoro-3-methoxy-indol-1-yl)-ethylamin fumarat (1:1) als beige Kristalle mit Smp. von 167°.

Beispiel 4

a) Eine Lösung von 1 g (6.6 mmol) 5-Chlorindol in 30 ml 1,2-Dibromethan wurde mit 30 ml 28%iger NaOH und 80 mg (0.24 mmol) Tetrabutylammoniumbromid versetzt. Das Gemisch wurde 3 Stunden bei 50° gerührt. Die Phasen wurden getrennt und die wässrige Phase wurde mit Toluol extrahiert. Die vereinigten organischen Phasen wurden mit Wasser gewaschen und über Natriumsulfat getrocknet. Das Lösungsmittel wurde abdestilliert und der Rückstand wurde über 150 g Silicagel mit Hexan-Essigester (5:1) chromatographiert. Man erhielt 1.17 g (69%) 1-(2-Bromethyl)-5-chlorindol als weisse Kristalle mit einem Smp. von 71°.

b) Eine Suspension von 0.55 g (2.1 mmol) 1-(2-Bromethyl)-5-chlorindol in 60 ml flüssigem Ammoniak wurde 18 Stunden bei 80° im Autoklaven gerührt. Nach dem Abdampfen des Ammoniaks wurde der Rückstand in Dichlormethan aufgenommen und mit Wasser und gesättigter Kochsalzlösung gewaschen. Die organische Phase wurde über Natriumsulfat getrocknet und das Lösungsmittel wurde abdestilliert. Der Rückstand wurde in 60 ml Ether und 3 ml Methanol gelöst und mit 0.25 g (2.1 mmol) Fumarsäure versetzt und 2 Tage gerührt. Die Kristalle wurden abfiltriert und getrocknet. Man erhielt 0.51 g (88 %) 2-(5-Chlorindol-1-yl)-ethylamin fumarat (1:0.7) als weisse Kristalle (Smp. 167°).

Beispiel 5

a) Zu einer Lösung von 145 ml (1.02 mol) 2-Methylacetessigester in 1 l Ethanol wurden bei 2-4° 400 ml 50%ige Kalilauge über einen Zeitraum von 90 min zugetropft. Es wurden 2 l Eiswasser zugegeben und schnell mit einer Diazoniumsalzlösung versetzt, die wie folgt bereitet wurde: Unter Eisbadkühlung wurden 200 ml einer 25%igen Salzsäurelösung zu einer Lösung von 145.6 g (1 mol) 3-Chlor-4-fluoranilin in 1 l Ethanol getropft. Anschliessend wurden bei 4° innerhalb von 90 min 137 ml (1.02 mol) Isopentylnitrit zugefügt. Die orange Emulsion, die aus der Zugabe der Diazoniumsalzlösung resultierte, wurde auf 4 l Wasser gegossen und einmal mit 4 l und zweimal mit je 2 l Toluol extrahiert. Die vereinigten organischen Phasen wurden über Natriumsulfat getrocknet, filtriert und auf ein Volumen von 1.5 l eingeengt. Eine Lösung von 203 g (1.07 mol) p-Toluolsulfonsäuremonohydrat in 1.5 l Toluol wurde, nachdem man 30 min am Wasserabscheider gekocht hat, zugefügt und man erhitzte die Mischung eine weitere Stunde am Wasserabscheider. Nach dem Abkühlen extrahierte man mit 1.5 l 1N Salzsäure, 1.5 l 1N Natronlauge und 0.4 l gesättigter Kochsalzlösung. Die Wasserphasen wurden mit 1.5 l Toluol nachgewaschen, und die vereinigten organischen Phasen wurden über Natriumsulfat getrocknet, filtriert

und eingedampft. Der Rückstand wurde über 3 kg Kieselgel mit Toluol chromatographiert. Man erhielt 10.3 g (4.2 %) rohen 6-Chlor-5-fluorindol-2-carbonsäure ethylester. Eine Probe wurde aus Toluol umkristallisiert und zeigte dann einen Smp. von 190-191°. Eine zweite Fraktion enthielt 7 g (2.9%) 4-Chlor-5-fluorindol-2-carbonsäure ethylester als braune Kristalle mit einem Smp. von 179-182°.

b) Eine Suspension von 6.3 g (26 mmol) 4-Chlor-5-fluorindol-2-carbonsäure ethylester in 260 ml Ethanol wurde mit 130 ml 2N Natronlauge versetzt und 3 Stunden gerührt. Ethanol wurde abgedampft und die Reaktionslösung wurde mit 25%iger Salzsäure auf pH 1 eingestellt. Der Niederschlag wurde mit Wasser gewaschen und getrocknet. Man erhielt 5.3 g (96%) rohe 4-Chlor-5-fluorindol-2-carbonsäure, die ohne weitere Reinigung in die nächste Stufe eingesetzt wurde.

c) Ein Metallbad wurde auf 300-320° geheitzt. Man brachte 4.8 g (22.47 mmol) 4-Chlor-5-fluorindol-2-carbonsäure unter Argon ein und nach drei Minuten wurde das Metallbad entfernt. Die Reaktionsmischung wurde im Kugelrohr bei 0.15 mbar und 75° Badtemperatur destilliert. Man erhielt 3.15 g (83%) 4-Chlor-5-fluorindol als weisse Kristalle mit Smp. 41-43°.

d) Eine Lösung von 130 mg (0.75 mmol) 4-Chlor-5-fluorindol in 3.7 ml 1,2-Dibromethan wurde mit 3.7 ml 28%iger NaOH und 7.7 mg (0.003 mmol) Tetrabutylammoniumbromid versetzt. Das Gemisch wurde 5 Stunden bei 50° gerührt. Die Phasen wurden getrennt und die wässrige Phase wurde mit Toluol extrahiert. Die vereinigten organischen Phasen wurden mit Wasser gewaschen und über Natriumsulfat getrocknet. Das Lösungsmittel wurde abdestilliert und der Rückstand wurde über 15 g Kieselgel mit Hexan-Essigester (5:1) chromatographiert. Man erhielt ein gelbes Öl, das in 30 ml flüssigem Ammoniak suspendiert und 16 Stunden bei 80° im Autoklaven gerührt wurde. Nach dem Abdampfen des Ammoniaks wurde der Rückstand in Dichlormethan aufgenommen und mit Wasser und gesättigter Kochsalzlösung gewaschen. Die organische Phase wurde über Natriumsulfat getrocknet und das Lösungsmittel wurde abdestilliert. Der Rückstand wurde über 15 g Kieselgel mit Essigester-Methanol (5:1) chromatographiert. Man erhielt 110 mg eines gelben Öls, das in 16 ml Ether gelöst, mit 60 mg (0.5 mmol) Fumarsäure versetzt und 2 Tage gerührt wurde. Die Kristalle wurden abfiltriert und getrocknet. Man erhielt 150 mg (59 %) 2-(4-Chlor-5-fluor-indol-1-yl)-ethylamin fumarat (1:1) mit Smp.200-201° (Zers.)

Beispiel 6

a) Eine Suspension von 21.8 g (90.2 mmol) 6-Chlor-5-fluorindol-2-carbonsäure ethylester in 450 ml Ethanol wurde mit 180 ml 2N Natronlauge versetzt und 21 Stunden gerührt. Die Lösung wurde eingedampft und in 450 ml Wasser aufgenommen und mit 60 ml 25%iger Salzsäure versetzt. Der Niederschlag wurde mit Wasser gewaschen und getrocknet. Man erhielt 18.8 g (97.7%) 6-Chlor-5-fluorindol-2-carbonsäure. Eine Probe wurde aus Toluol umkristallisiert und zeigte dann einen Smp. von 274-276°.

b) Ein Metallbad wurde auf 300-320° geheitzt. Man brachte 6.4 g (30 mmol) 6-Chlor-5-fluorindol-2-carbonsäure unter Argon ein und nach drei Minuten wurde das Metallbad entfernt. Nach dem Abkühlen wurde die Reaktionsmischung über 25 g Kieselgel mit Hexan-Essigester (4:1) chromatographiert. Man erhielt 4.17 g (85 %) 6- Chlor-5-fluorindol als beige Kristalle. Eine Probe wurde mit Hexan verrührt und zeigte dann einen Smp. von 96-98°.

c) Eine Lösung von 0.95g (5.6 mmol) 6-Chlor-5-fluorindol in 30 ml 1,2-Dibromethan wurde mit 30 ml 28%iger NaOH und 60 mg (0.18 mmol) Tetrabutylammoniumbromid versetzt. Das Gemisch wurde 8 Stunden gerührt. Die Phasen wurden getrennt und die wässrige Phase wurde mit Toluol extrahiert. Die vereinigten organischen Phasen wurden mit Wasser gewaschen und über Natriumsulfat getrocknet. Das Lösungsmittel wurde abdestilliert und der Rückstand wurde über 100 g Kieselgel mit Hexan-Essigester (6:1) chromatographiert. Man erhielt ein hellbraunes Öl, das in 120 ml flüssigem Ammoniak suspendiert und 16 Stunden bei 80° im Autoklaven gerührt wurde. Nach dem Abdampfen des Ammoniaks wurde der Rückstand in Dichlormethan aufgenommen und mit Wasser und gesättigter Kochsalzlösung gewaschen. Die organische Phase wurde über Natriumsulfat getrocknet und das Lösungsmittel wurde abdestilliert. Der Rückstand wurde in 120 ml Ether und 6 ml Methanol gelöst, mit 0.5 g (4.3 mmol) Fumarsäure versetzt und über Nacht gerührt. Die Kristalle wurden abfiltriert und getrocknet. Man erhielt 1 g (61 %) 2-(6-Chlor-5-fluor-indol-1-yl)-ethylamin fumarat (1:0.7) mit Smp.192-193° (Zers.)

Beispiel 7

a) Eine Lösung von 1.76 g (7.54 mmol) 4-Methyl-3-methoxyindol-2-carbonsäure ethylester in 90 ml Ethanol wurden mit 45 ml 2N Natronlauge versetzt und 17 Stunden bei Raumtemperatur gerührt. Man

dampfte den Alkohol ab und versetzte den Rückstand mit 60 ml 2N Salzsäure. Die ausgefallenen Kristalle wurden abfiltriert mit Wasser gewaschen und getrocknet. Man erhielt 1.2 g (78 %) 4-Methyl-3-methoxyindol-2-carbonsäure als braune Kristalle mit Smp. 136°.

b) Bei 150° wurden 1.2 g (5.85 mmol) 4-Methyl-3-methoxyindol-2-carbonsäuresolange erhitzt bis kein Gas mehr entwich. Man erhielt 0.94 g (quant.) rohes 4-Methyl-3-methoxyindol, das ohne weitere Reinigung in die nächste Stufe eingesetzt wurde.

c) Eine Lösung von 0.94 g (5.8 mmol) 4-Methyl-3-methoxyindol in 30 ml 1,2-Dibromethan wurde mit 30 ml 28%iger NaOH und 400 mg (1.2 mmol) Tetrabutylammoniumbromid versetzt. Das Gemisch wurde 24 Stunden bei 40° gerührt. Das Reaktionsgemisch wurde mit 150 ml Toluol verdünnt und mit 50 ml Wasser und 25 ml gesättigter Kochsalzlösung gewaschen. Die Wasserphasen wurden mit 75 ml Toluol nachextrahiert. Die vereinigten organischen Phasen wurden mit Natriumsulfat getrocknet. Das Lösungsmittel wurde abdestilliert und der Rückstand wurde über 70 g Kieselgel mit Hexan-Essigester (6:1) chromatographiert. Man erhielt ein rotes Öl, das in 20 ml flüssigem Ammoniak suspendiert und 16 Stunden bei 80° im Autoklaven gerührt wurde. Nach dem Abdampfen des Ammoniaks wurde der Rückstand in Dichlormethan aufgenommen und mit Wasser und gesättigter Kochsalzlösung gewaschen. Die organische Phase wurde über Natriumsulfat getrocknet und das Lösungsmittel wurde abdestilliert. Der Rückstand wurde in 60 ml Ether gelöst und mit 0.3 g (2.5 mmol) Fumarsäure versetzt und 18 Stunden gerührt. Die ausgefallenen Kristalle wurden abfiltriert und getrocknet. Man erhielt 0.5 g (28%) 2-(4-Methyl-3-methoxy-indol-1-yl)-ethylamin fumarat (1:0.9) mit Smp.163-164°.

Beispiel 8

a) Eine Suspension von 0.4 g (14.3 mmol, 80%) Natriumhydrid-Dispersion in 60 ml Tetrahydrofuran wurde bei 0° mit 1.74 g (11.5 mmol) 5-Chlorindol versetzt und 1 Stunde bei dieser Temperatur gerührt. Nach der Zugabe von 1.6 ml (23 mmol) (RS)-Methyloxiran wurde das Reaktionsgemisch 48 Stunden bei Raumtemperatur gerührt und anschliessend mit 11 ml Wasser versetzt. Es wurde mit 300 ml Ether verdünnt, mit 140 ml Wasser und mit 70 ml gesättigter Kochsalzlösung gewaschen und die organische Phase über Natriumsulfat getrocknet. Nach dem Entfernen des Lösungsmittels wurde der Rückstand über 60 g Kieselgel mit Toluol-Essigester (19:1) chromatographiert. Man erhielt 2.1 g (87%) (RS)-1-(5-Chlor-indol-1-yl)-propan-2-ol als gelbes Öl.
MS: m/e (%Basispeak): 209, 211 ($M^+$,28), 164 (100)

b) Eine Lösung von 2 g (9.6 mmol) (RS)-1-(5-Chlor-indol-1-yl)-propan-2-ol in 50 ml Dichlormethan wurde mit 5.4 ml (38,7 mmol) Triethylamin versetzt und auf 0° gekühlt. Zu dieser Lösung wurde 1.5 ml (19.3 mmol) Methansulfonsäurechlorid zugetropft und das Reaktionsgemisch wurde eine Stunde gerührt. Man verdünnte mit 480 ml Ether. Es wurde mit 120 ml 1M Natriumcarbonatlösung und 60 ml gesättigter Kochsalzlösung gewaschen und die wässrige Phase mit 240 ml Ether nachextrahiert. Die vereinigten organischen Phasen wurden über Natriumsulfat getrocknet und das Lösungsmittel wurde entfernt. Der Rückstand wurde in 50 ml Dimethylformamid gelöst und nach der Zugabe von 1.26 g (19.4 mmol) Natriumazid 7 Stunden bei 60° gerührt Man verdünnte mit 500 ml Ether und extrahierte zweimal mit je 240 ml Wasser und einmal mit 60 ml gesättigter Kochsalzlösung. Die Wasserphase wurde mit 240 ml Ether nachextrahiert und die vereinigten organischen Phasen wurden über Natriumsulfat getrocknet. Das Lösungsmittel wurde entfernt und der Rückstand über 46 g Kieselgel mit Toluol chromatographiert. Man erhielt 2 g (90%) (RS)-1-(2-Azido-propyl)-5-chlorindol als farbloses Öl.
MS: m/e (%Basispeak): 234, 236 ($M^+$,20), 164 (100)

c) Eine Suspension von 0.2 g Platinoxid in 40 ml Ethanol wurde eine halbe Stunde unter Wasserstoffatmosphäre gerührt und anschliessend mit einer Lösung von 1.9 g (8.1 mmol) (RS)-1-(2-Azido-propyl)-5-chlorindol in 40 ml Ethanol versetzt. Das Reaktionsgemisch wurde zwei Stunden bei Raumtemperatur gerührt. Der Katalysator wurde abfiltriert und mit Ethanol gewaschen. Die Lösung wurde eingedampft und der Rückstand wurde in 160 ml Ether gelöst und mit 0.94 g (8.1 mmol) Fumarsäure versetzt und über Nacht gerührt. Die ausgefallenen Kristalle wurden abfiltriert und getrocknet. Man erhielt 1.85 g (70%) (RS)-2-(5-Chlor-indol-1-yl)-1-methyl-ethylamin fumarat (1:1.9) als weisse Kristalle mit Smp. 183-185° (Zers.)

Beispiel 9

a) Eine Suspension von 0.55 g (18.5 mmol, 80%) Natriumhydrid-Dispersion in 75 ml Tetrahydrofuran wurde bei 0° mit 2 g (14.8 mmol) 5-Fluorindol versetzt und 1 Stunde bei dieser Temperatur gerührt. Nach der Zugabe von 2.1 ml (30 mmol) (RS)-Methyloxiran wurde das Reaktionsgemisch 24 Stunden bei

Raumtemperatur gerührt und anschliessend mit 5 ml Wasser versetzt. Es wurde mit Ether verdünnt, dreimal mit je 75 ml Wasser und mit 70 ml gesättigter Kochsalzlösung gewaschen und die organische Phase über Natriumsulfat getrocknet. Nach dem Entfernen des Lösungsmittels wurde der Rückstand über 60 g Kieselgel mit Toluol-Essigester (19:1) chromatographiert. Man erhielt 1.8 g (62%) (RS)-1-(5-Fluor-indol-1-yl)-propan-2-ol als gelbes Öl.

MS: m/e (%Basispeak): 193 ($M^+$,22), 148 (100)

b) Eine Lösung von 1.75 g (9 mmol) (RS)-1-(5-Fluor-indol-1-yl)-propan-2-ol in 45 ml Dichlormethan wurde mit 3.8 ml (27 mmol) Triethylamin versetzt und auf 0° gekühlt. Zu dieser Lösung wurde 1.4 ml (18 mmol) Methansulfonsäurechlorid zugetropft und das Reaktionsgemisch wurde eine Stunde gerührt. Man verdünnte mit Ether. Es wurde mit 1M Natriumcarbonatlösung und gesättigter Kochsalzlösung gewaschen und die wässrige Phase mit Ether nachextrahiert. Die vereinigten organischen Phasen wurden über Natriumsulfat getrocknet und das Lösungsmittel wurde entfernt. Der Rückstand wurde in 45 ml Dimethylformamid gelöst und nach der Zugabe von 1.18 g (18.1 mmol) Natriumazid 6 Stunden bei 60° gerührt. Man verdünnte mit Ether und extrahierte mit Wasser und mit 60 ml gesättigter Kochsalzlösung. Die Wasserphase wurde mit Ether nachextrahiert und die vereinigten organischen Phasen wurden über Natriumsulfat getrocknet. Das Lösungsmittel wurde entfernt und der Rückstand über 20 g Kieslgel mit Toluol chromatographiert. Man erhielt 1.8 g (91%) (RS)-1-(2-Azido-propyl)-5-fluorindol als farbloses Öl.

MS: m/e (%Basispeak): 218 ($M^+$,19), 148 (100)

c) Eine Lösung von 1.7 g (7,7 mmol) (RS)-1-(2-Azido-propyl)-5-fluorindol in 80 ml Ethanol wurde über 170 mg Pd-C (5%) hydriert. Der Katalysator wurde abfiltriert und mit Ethanol gewaschen. Die Lösung wurde eingedampft und der Rückstand wurde in 240 ml Ether gelöst und mit 0.93 g (8 mmol) Fumarsäure versetzt und über Nacht gerührt. Die ausgefallenen Kristalle wurden abfiltriert und getrocknet. Man erhielt 2.3 g (95.8%) (RS)-2-(5-Fluor-indol-1-yl)-1-methyl-ethylamin fumarat (1:1) als weisse Kristalle mit Smp. 169-170° (Zers.)

## Beispiel 10

a) Eine Suspension von 0.75 g (24 mmol, 80%) Natriumhydrid-Dispersion in 100 ml Tetrahydrofuran wurde bei 0° mit 3.38 g (20 mmol) 6-Chlor-5-fluorindol versetzt und 1 Stunde bei dieser Temperatur gerührt. Nach der Zugabe von 2.8 ml (40 mmol) (RS)-Methyloxiran wurde das Reaktionsgemisch 48 Stunden bei Raumtemperatur gerührt und anschliessend mit 11 ml Wasser versetzt. Es wurde mit Ether verdünnt, mit Wasser und mit gesättigter Kochsalzlösung gewaschen und die organische Phase über Natriumsulfat getrocknet. Nach dem Entfernen des Lösungsmittels wurde der Rückstand über 75 g Kieselgel mit Toluol-Essigester (19:1) chromatographiert. Man erhielt 2.87 g (63%) (RS)-1-6-Chlor-5-fluor-indol-1-yl)-propan-2-ol als beige Kristalle mit Smp. 185-186°.

b) Eine Lösung von 2.85 g (12.5 mmol) (RS)-1-(6-Chlor-5-fluor-indol-1-yl)-propan-2-ol in 60 ml Dichlormethan wurde mit 5.2 ml (37.6 mmol) Triethylamin versetzt und auf 0° gekühlt. Zu dieser Lösung wurde 1.9 ml (25.1 mmol) Methansulfonsäurechlorid zugetropft und das Reaktionsgemisch wurde eine Stunde gerührt. Man verdünnte mit Ether. Es wurde mit 1M Natriumcarbonatlösung und gesättigter Kochsalzlösung gewaschen und die wässrige Phase mit Ether nachextrahiert. Die vereinigten organischen Phasen wurden über Natriumsulfat getrocknet und das Lösungsmittel wurde entfernt. Der Rückstand wurde in 60 ml Dimethylformamid gelöst und nach der Zugabe von 1.55 g (23.8 mmol) Natriumazid 3 Stunden bei 60° gerührt. Man verdünnte mit Ether und extrahierte zweimal mit Wasser und mit gesättigter Kochsalzlösung. Die Wasserphase wurde mit Ether nachextrahiert und die vereinigten organischen Phasen wurden über Natriumsulfat getrocknet. Das Lösungsmittel wurde entfernt und der Rückstand über 60 g Kieselgel mit Toluol chromatographiert. Man erhielt 2.87 g (92%) (RS)-1-(2-Azido-propyl)-6-chlor-5-fluorindol als gelbliches Öl.

MS: m/e (%Basispeak): 252, 254 ($M^+$,20), 182 (100)

c) Eine Suspension von 0.26 g Platinoxid in 50 ml Ethanol wurde eine halbe Stunde unter Wasserstoffatmosphäre gerührt und anschliessend mit einer Lösung von 2.8 g (11.7 mmol) (RS)-1-(2-Azido-propyl)-6-chlor-5-fluorindol in 50 ml Ethanol versetzt. Das Reaktionsgemisch wurde zwei Stunden bei Raumtemperatur gerührt. Der Katalysator wurde abfiltriert und mit Ethanol gewaschen. Die Lösung wurde eingedampft und der Rückstand wurde in 380 ml Ether gelöst und mit 1.28 g (11 mmol) Fumarsäure versetzt und über Nacht gerührt. Die ausgefallenen Kristalle wurden abfiltriert und getrocknet. Man erhielt 3.6 g (95%) (RS)-2-(6-Chlor-5-fluor-indol-1-yl)-1-methyl-ethylamin fumarat (1:1) als weisse Kristalle mit Smp. 174-175° (Zers.)

Beispiel 11

a) Eine Suspension von 0.26 g (8.75 mmol, 80%) Natriumhydrid-Dispersion in 35 ml Tetrahydrofuran wurde bei 0° mit 0.95 g (7 mmol) 5-Fluorindol versetzt und 1 Stunde bei dieser Temperatur gerührt. Nach der Zugabe von 1 ml (14 mmol) (S)-Methyloxiran wurde das Reaktionsgemisch 48 Stunden bei Raumtemperatur gerührt und anschliessend mit 7 ml Wasser versetzt. Es wurde mit 180 ml Ether verdünnt, zweimal mit je 90 ml Wasser und mit 50 ml gesättigter Kochsalzlösung gewaschen und die organische Phase über Natriumsulfat getrocknet. Nach dem Entfernen des Lösungsmittels wurde der Rückstand über 28 g Kieselgel mit Toluol-Essigester (19:1) chromatographiert. Man erhielt 1.15 g (84%) (S)-1-(5-Fluor-indol-1-yl)-propan-2-ol als hellbraunes Öl.
$[\alpha]_D^{20}$ = +48.4 (c 0.25, $CHCl_3$)
b) Eine Lösung von 1.1 g (5.7 mmol) (S)-1-(5-Fluor-indol-1-yl)-propan-2-ol in 30 ml Dichlormethan wurde mit 3.17 ml (38,7 mmol) Triethylamin versetzt und auf 0° gekühlt. Zu dieser Lösung wurde 0.88 ml (11.4 mmol) Methansulfonsäurechlorid zugetropft und das Reaktionsgemisch wurde eine Stunde gerührt. Man verdünnte mit 280 ml Ether. Es wurde zweimal mit je 70 ml 1M Natriumcarbonatlösung und 35 ml gesättigter Kochsalzlösung gewaschen und die wässrige Phase mit 140 ml Ether nachextrahiert. Die vereinigten organischen Phasen wurden über Natriumsulfat getrocknet und das Lösungsmittel wurde entfernt. Der Rückstand wurde in 30 ml Dimethylformamid gelöst und nach der Zugabe von 0.74 g (11.4 mmol) Natriumazid 7 Stunden bei 60° gerührt. Man verdünnte mit 280 ml Ether und extrahierte zweimal mit je 140 ml Wasser und einmal mit 70 ml gesättigter Kochsalzlösung. Die Wasserphase wurde mit 140 ml Ether nachextrahiert und die vereinigten organischen Phasen wurden über Natriumsulfat getrocknet. Das Lösungsmittel wurde entfernt und der Rückstand über 25 g Kieselgel mit Toluol chromatographiert. Man erhielt 1.14 g (95%) (R)-1-(2-Azido-propyl)-5-fluorindol als gelbliches Öl.
$[\alpha]_D^{20}$ = -124 (c 0.25, $CHCl_3$)
c) Eine Suspension von 0.1 g Platinoxid in 25 ml Ethanol wurde eine halbe Stunde unter Wasserstoffatmosphäre gerührt und anschliessend mit einer Lösung von 0.92 g (4.8 mmol) (R)-1-(2-Azido-propyl)-5-fluorindol in 25 ml Ethanol versetzt. Das Reaktionsgemisch wurde zwei Stunden bei Raumtemperatur gerührt. Der Katalysator wurde abfiltriert und mit Ethanol gewaschen. Die Lösung wurde eingedampft und der Rückstand wurde in 150 ml Ether gelöst und mit 0.58 g (5 mmol) Fumarsäure versetzt und über Nacht gerührt. Die ausgefallenen Kristalle wurden abfiltriert und getrocknet. Man erhielt 1.14 g (88%) (R)-2-(5-Fluor-indol-1-yl)-1-methyl-ethylamin fumarat (1:1) als weisse Kristalle mit Smp. 159-161° (Zers.)
$[\alpha]_D^{20}$ = -38 (c 0.25, $CH_3OH$)

Beispiel 12

a) Eine Suspension von 0.26 g (8.75 mmol, 80%) Natriumhydrid-Dispersion in 35 ml Tetrahydrofuran wurde bei 0° mit 0.95 g (7 mmol) 5-Fluorindol versetzt und 1 Stunde bei dieser Temperatur gerührt. Nach der Zugabe von 1 ml (14 mmol) (R)-Methyloxiran wurde das Reaktionsgemisch 48 Stunden bei Raumtemperatur gerührt und anschliessend mit 7 ml Wasser versetzt. Es wurde mit 180 ml Ether verdünnt, zweimal mit je 90 ml Wasser und mit 50 ml gesättigter Kochsalzlösung gewaschen und die organische Phase über Natriumsulfat getrocknet. Nach dem Entfernen des Lösungsmittels wurde der Rückstand über 28 g Kieselgel mit Toluol-Essigester (19:1) chromatographiert. Man erhielt 1.09 g (80%) (R)-1-(5-Fluor-indol-1-yl)-propan-2-ol als hellbraunes Öl.
$[\alpha]_D^{20}$ = -48.8 (c 0.25, $CHCl_3$)
b) Eine Lösung von 1.04 g (5.3 mmol) (R)-1-(5-Fluor-indol-1-yl)-propan-2-ol in 30 ml Dichlormethan wurde mit 3.17 ml (38,7 mmol) Triethylamin versetzt und auf 0° gekühlt. Zu dieser Lösung wurde 0.88 ml (11.4 mmol) Methansulfonsäurechlorid zugetropft und das Reaktionsgemisch wurde eine Stunde gerührt. Man verdünnte mit 280 ml Ether. Es wurde zweimal mit je 70 ml 1M Natriumcarbonatlösung und 35 ml gesättigter Kochsalzlösung gewaschen und die wässrige Phase mit 140 ml Ether nachextrahiert. Die vereinigten organischen Phasen wurden über Natriumsulfat getrocknet und das Lösungsmittel wurde entfernt. Der Rückstand wurde in 30 ml Dimethylformamid gelöst und nach der Zugabe von 0.74 g (11.4 mmol) Natriumazid 7 Stunden bei 60° gerührt. Man verdünnte mit 280 ml Ether und extrahierte zweimal mit je 140 ml Wasser und einmal mit 70 ml gesättigter Kochsalzlösung. Die Wasserphase wurde mit 140 ml Ether nachextrahiert und die vereinigten organischen Phasen wurden über Natriumsulfat getrocknet. Das Lösungsmittel wurde entfernt und der Rückstand über 25 g Kieslgel mit Toluol chromatographiert. Man erhielt 1.14 g (97.4%) (S)-1-(2-Azido-propyl)-5-fluorindol als gelbliches Öl.
$[\alpha]_D^{20}$ = -119.2 (c 0.25, $CHCl_3$)

c) Eine Suspension von 0.1 g Platinoxid in 25 ml Ethanol wurde eine halbe Stunde unter Wasserstoffatmosphäre gerührt und anschliessend mit einer Lösung von 1.12 g (5.13 mmol) (S)-1-(2-Azido-propyl)-5-fluorindol in 25 ml Ethanol versetzt. Das Reaktionsgemisch wurde zwei Stunden bei Raumtemperatur gerührt. Der Katalysator wurde abfiltriert und mit Ethanol gewaschen. Die Lösung wurde eingedampft und der Rückstand wurde in 150 ml Ether gelöst und mit 0.58 g (5 mmol) Fumarsäure versetzt und über Nacht gerührt. Die ausgefallenen Kristalle wurden abfiltriert und getrocknet. Man erhielt 1.42 g (90%) (S)-2-(6-Chlor-5-fluor-indol-1-yl)-1-methyl-ethylamin fumarat (1:1) als weisse Kristalle mit Smp. 159-161° (Zers.)

$[\alpha]_D^{20}$ = -34 (c 0.25, $CH_3OH$)

Beispiel 13

a) Eine Suspension von 0.11 g (3.7 mmol, 80%) Natriumhydrid-Dispersion in 15 ml Tetrahydrofuran wurde bei 0° mit 0.5 g (3 mmol) 6-Chlor-5-fluorindol versetzt und 1 Stunde bei dieser Temperatur gerührt. Nach der Zugabe von 0.42 ml (6 mmol) (S)-Methyloxiran wurde das Reaktionsgemisch 85 Stunden bei Raumtemperatur gerührt und anschliessend mit Wasser versetzt. Es wurde mit Ether verdünnt, mit Wasser und mit gesättigter Kochsalzlösung gewaschen und die organische Phase über Natriumsulfat getrocknet. Nach dem Entfernen des Lösungsmittels wurde der Rückstand über 25 g Kieselgel mit Toluol-Essigester (19:1) chromatographiert. Man erhielt 0.54 g (79%) (S)-1-(6-chlor-5-luor-indol-1-yl)-propan-2-ol als beige Kristalle (Smp. 99-101°).

b) Eine Lösung von 0.53 g (2.3 mmol) (S)-1-(6-Chlor-5-fluor-indol-1-yl)-propan-2-ol in 30 ml Dichlormethan wurde mit 1 ml (7 mmol) Triethylamin versetzt und auf 0° gekühlt. Zu dieser Lösung wurde 0.36 ml (4.6 mmol) Methansulfonsäurechlorid zugetropft und das Reaktionsgemisch wurde eine Stunde gerührt. Man verdünnte mit Ether. Es wurde mit 1M Natriumcarbonatlösung und gesättigter Kochsalzlösung gewaschen und die wässrige Phase mit Ether nachextrahiert. Die vereinigten organischen Phasen wurden über Natriumsulfat getrocknet und das Lösungsmittel wurde entfernt. Der Rückstand wurde in 10 ml Dimethylformamid gelöst und nach der Zugabe von 0.3 g (4.6 mmol) Natriumazid 7 Stunden bei 60° gerührt Man verdünnte mit Ether und extrahierte mit Wasser und mit gesättigter Kochsalzlösung. Die Wasserphase wurde mit Ether nachextrahiert und die vereinigten organischen Phasen wurden über Natriumsulfat getrocknet. Das Lösungsmittel wurde entfernt und der Rückstand über 20 g Kieslgel mit Toluol-n-Hexan (9:1) chromatographiert. Man erhielt 0.54 g (92%) (R)-1-(2-Azido-propyl)-6-chlor-5-fluorindol als gelbliches Öl.

$[\alpha]_D^{20}$ = -131.6 (c 0.25, $CHCl_3$)

c) Eine Suspension von 0.05 g Platinoxid in 10 ml Ethanol wurde eine halbe Stunde unter Wasserstoffatmosphäre gerührt und anschliessend mit einer Lösung von 0.51 g (2 mmol) (R)-1-(2-Azido-propyl)-6-chlor-5-fluorindol in 10 ml Ethanol versetzt. Das Reaktionsgemisch wurde zwei Stunden bei Raumtemperatur gerührt. Der Katalysator wurde abfiltriert und mit Ethanol gewaschen. Die Lösung wurde eingedampft und der Rückstand wurde in 60 ml Ether gelöst und mit 0.23 g (1.98 mmol) Fumarsäure versetzt und über Nacht gerührt. Die ausgefallenen Kristalle wurden abfiltriert und getrocknet. Man erhielt 0.55 g (69%) (R)-2-(6-Chlor-5-fluor-indol-1-yl)-1-methyl-ethylamin fumarat (1:1.5) als weisse Kristalle mit Smp. 153-154° (Zers.)

$[\alpha]_D^{20}$ = -28.8 (c 0.25, $CH_3OH$)

Beispiel 14

a) Eine Suspension von 0,11 g (3.7 mmol, 80%) Natriumhydrid-Dispersion in 15 ml Tetrahydrofuran wurde bei 0° mit 0.5 g (3 mmol) 6-Chlor-5-fluorindol versetzt und 1 Stunde bei dieser Temperatur gerührt. Nach der Zugabe von 0.42 ml (6 mmol) (R)-Methyloxiran wurde das Reaktionsgemisch 85 Stunden bei Raumtemperatur gerührt und anschliessend mit Wasser versetzt. Es wurde mit Ether verdünnt, mit Wasser und mit gesättigter Kochsalzlösung gewaschen und die organische Phase über Natriumsulfat getrocknet. Nach dem Entfernen des Lösungsmittels wurde der Rückstand über 25 g Kieselgel mit Toluol-Essigester (19:1) chromatographiert. Man erhielt 0.51 g (74.6%) (R)-1-(6-chlor-5-fluor-indol-1-yl)-propan-2-ol als weisse Kristalle (Smp. 104-105°.)

b) Eine Lösung von 0.28 g (1.2 mmol) (R)-1-(6-Chlor-5-fluor-indol-1-yl)-propan-2-ol in 6 ml Dichlormethan wurde mit 0.5 ml (3.8 mmol) Triethylamin versetzt und auf 0° gekühlt. Zu dieser Lösung wurde 0.2 ml (2.5 mmol) Methansulfonsäurechlorid zugetropft und das Reaktionsgemisch wurde eine Stunde gerührt. Man verdünnte mit Ether. Es wurde mit 1M Natriumcarbonatlösung und gesättigter Kochsalzlösung gewaschen und die wässrige Phase mit Ether nachextrahiert. Die vereinigten organischen Phasen

wurden über Natriumsulfat getrocknet und das Lösungsmittel wurde entfernt. Der Rückstand wurde in 6 ml Dimethylformamid gelöst und nach der Zugabe von 0.16 g (2.4 mmol) Natriumazid 7 Stunden bei 60° gerührt. Man verdünnte mit Ether und extrahierte mit Wasser und mit gesättigter Kochsalzlösung. Die Wasserphase wurde mit Ether nachextrahiert und die vereinigten organischen Phasen wurden über Natriumsulfat getrocknet. Das Lösungsmittel wurde entfernt und der Rückstand über 10 g Kieselgel mit Toluol chromatographiert. Man erhielt 0.29 g (93.5%) (S)-1-(2-Azido-propyl)-6-chlor-5-fluorindol als gelbliches Öl.

$[\alpha]_D^{20}$ = +151.2 (c 0.25, $CHCl_3$)

c) Eine Suspension von 0.02 g Platinoxid in 5 ml Ethanol wurde eine halbe Stunde unter Wasserstoffatmosphäre gerührt und anschliessend mit einer Lösung von 0.26 g (1.05 mmol) (S)-1-(2-Azido-propyl)-6-chlor-5-fluorindol in 5 ml Ethanol versetzt. Das Reaktionsgemisch wurde zwei Stunden bei Raumtemperatur gerührt. Der Katalysator wurde abfiltriert und mit Ethanol gewaschen. Die Lösung wurde eingedampft und der Rückstand wurde in 30 ml Ether gelöst und mit 0.12 g (1.03 mmol) Fumarsäure versetzt und über Nacht gerührt. Die ausgefallenen Kristalle wurden abfiltriert und getrocknet. Man erhielt 0.25 g (58.8%) (S)-2-(6-Chlor-5-fluor-indol-1-yl)-1-methyl-ethylamin fumarat (1:1.6) als weisse Kristalle mit Smp. 151-152° (Zers.)

$[\alpha]_D^{20}$ = +31.6 (c 0.25, $CH_3OH$)

Beispiel 15

a) Eine Suspension von 0.26 g (8.7 mmol, 80%) Natriumhydrid-Dispersion in 35 ml Tetrahydrofuran wurde bei 0° mit 0.95 g (7.25 mmol) 4-Methylindol versetzt und 1 Stunde bei dieser Temperatur gerührt. Nach der Zugabe von 1 ml (15 mmol) (RS)-Methyloxiran wurde das Reaktionsgemisch 48 Stunden bei Raumtemperatur gerührt und anschliessend mit Wasser versetzt. Es wurde mit Ether verdünnt, mit Wasser und mit gesättigter Kochsalzlösung gewaschen und die organische Phase über Natriumsulfat getrocknet. Nach dem Entfernen des Lösungsmittels wurde der Rückstand über 25 g Kieselgel mit Toluol-Essigester (19:1) chromatographiert. Man erhielt 0.86 g (62.7%) (RS)-1-(4-Methyl-indol-1-yl)-propan-2-ol als gelbes Öl.

MS: m/e (%Basispeak): 189 ($M^+$,26), 144 (100)

b) Eine Lösung von 0.74 g (4 mmol) (RS)-1-(4-Methyl-indol-1-yl)-propan-2-ol in 20 ml Dichlormethan wurde mit 1.6 ml (11 mmol) Triethylamin versetzt und auf 0° gekühlt. Zu dieser Lösung wurde 0.6 ml (7.7 mmol) Methansulfonsäurechlorid zugetropft und das Reaktionsgemisch wurde eine Stunde gerührt. Man verdünnte mit Ether. Es wurde mit 1M Natriumcarbonatlösung und gesättigter Kochsalzlösung gewaschen und die wässrige Phase mit Ether nachextrahiert. Die vereinigten organischen Phasen wurden über Natriumsulfat getrocknet und das Lösungsmittel wurde entfernt. Der Rückstand wurde in 20 ml Dimethylformamid gelöst und nach der Zugabe von 0.5 g (7.8 mmol) Natriumazid 7 Stunden bei 60° gerührt. Man verdünnte mit Ether und extrahierte mit Wasser und gesättigter Kochsalzlösung. Die Wasserphase wurde mit Ether nachextrahiert und die vereinigten organischen Phasen wurden über Natriumsulfat getrocknet. Das Lösungsmittel wurde entfernt und der Rückstand über 30 g Kieselgel mit Toluol chromatographiert. Man erhielt 0.68 g (81.2%) (S)-1-(2-Azido-propyl)-4-methylindol als oranges Öl.

MS: m/e (%Basispeak): 214 ($M^+$,20), 144 (100)

c) Eine Lösung von 0.67 g (3 mmol) (RS)-1-(2-Azido-propyl)-4-methylindol in 30 ml Ethanol wurde über 70 mg Pd-C (5%) hydriert. Der Katalysator wurde abfiltriert und mit Ethanol gewaschen. Die Lösung wurde eingedampft und der Rückstand wurde in 90 ml Ether gelöst und mit 0.35 g (3 mmol) Fumarsäure versetzt und über Nacht gerührt. Die ausgefallenen Kristalle wurden abfiltriert und getrocknet. Man erhielt 0.88 g (92.3%) (RS)-2-(4-Methyl-indol-1-yl)-1-methyl-ethylamin fumarat (1:1) als weisse Kristalle mit Smp. 163-164° (Zers.)

Beispiel 16

a) Eine Suspension von 0,56 g (18.75 mmol, 80%) Natriumhydrid-Dispersion in 75 ml Tetrahydrofuran wurde bei 0° mit 2.95 g (15 mmol) 5-Bromindol versetzt und 1 Stunde bei dieser Temperatur gerührt. Nach der Zugabe von 2.1 ml (30 mmol) (RS)-Methyloxiran wurde das Reaktionsgemisch 60 Stunden bei Raumtemperatur gerührt und anschliessend mit 15 ml Wasser versetzt. Es wurde mit 750 ml Ether verdünnt, zweimal mit je 250 ml Wasser und mit 125 ml gesättigter Kochsalzlösung gewaschen und die organische Phase über Natriumsulfat getrocknet. Nach dem Entfernen des Lösungsmittels wurde der Rückstand über 120 g Kieselgel mit Toluol-Essigester (19:1) chromatographiert. Man erhielt 3.4 g (89%) (RS)-1-(5-Brom-indol-1-yl)-propan-2-ol als gelbes Öl.

MS: m/e (%Basispeak): 253, 255 ($M^+$,41), 208, 210 (100), 129 (70)

b) Eine Lösung von 3.36 g (13.2 mmol) (RS)-1-(5-Brom-indol-1-yl)-propan-2-ol in 60 ml Dichlormethan wurde mit 7.37 ml (53 mmol) Triethylamin versetzt und auf 0° gekühlt. Zu dieser Lösung wurden 2 ml (26.4 mmol) Methansulfonsäurechlorid zugetropft und das Reaktionsgemisch wurde eine Stunde gerührt. Man verdünnte mit 660 ml Ether. Es wurde zweimal mit je 135 ml 1M Natriumcarbonatlösung und 80 ml gesättigter Kochsalzlösung gewaschen und die wässrige Phase mit 300 ml Ether nachextrahiert. Die vereinigten organischen Phasen wurden über Natriumsulfat getrocknet und das Lösungsmittel wurde entfernt. Der Rückstand wurde in 60 ml Dimethylformamid gelöst und nach der Zugabe von 1.72 g (11.4 mmol) Natriumazid 16 Stunden bei 60° gerührt. Man verdünnte mit 660 ml Ether und extrahierte zweimal mit je 330 ml Wasser und einmal mit 80 ml gesättigter Kochsalzlösung. Die Wasserphase wurde mit 330 ml Ether nachextrahiert und die vereinigten organischen Phasen wurden über Natriumsulfat getrocknet. Das Lösungsmittel wurde entfernt und der Rückstand über 90 g Kieslgel mit Toluol chromatographiert. Man erhielt 3.22 g (87.2%) (RS)-1-(2-Azido-propyl)-5-bromindol als hellgelbes Öl.

MS: m/e (%Basispeak): 278, 280 ($M^+$,16), 208, 210 (87), 129 (100)

c) Eine Suspension von 0.1 g Platinoxid in 20 ml Ethanol wurde eine halbe Stunde unter Wasserstoffatmosphäre gerührt und anschliessend mit einer Lösung von 1.12 g (4 mmol) (RS)-1-(2-Azido-propyl)-5-bromindol in 20 ml Ethanol und 2 ml einer 33%igen Methylaminlösung in Ethanol versetzt. Das Reaktionsgemisch wurde 4 Stunden bei Raumtemperatur gerührt. Der Katalysator wurde abfiltriert und mit Ethanol gewaschen. Die Lösung wurde eingedampft und der Rückstand wurde in 120 ml Ether und 2.5 ml Methanol gelöst und mit 0.46 g (3.96 mmol) Fumarsäure versetzt und über Nacht gerührt. Die ausgefallenen Kristalle wurden abfiltriert und getrocknet. Man erhielt 1.38 g (93%) (RS)-2-(5-Brom-indol-1-yl)-1-methyl-ethylamin fumarat (1:1) als weisse Kristalle mit Smp. 192-193° (Zers.)

Beispiel 17

a) Eine Suspension von 0.28 g (9.35 mmol, 80%) Natriumhydrid-Dispersion in 40 ml Tetrahydrofuran wurde bei 0° mit 0.98 g (7.5 mmol) 6-Methylindol versetzt und 1 Stunde bei dieser Temperatur gerührt. Nach der Zugabe von 1 ml (15 mmol) (RS)-Methyloxiran wurde das Reaktionsgemisch 60 Stunden bei Raumtemperatur gerührt und anschliessend mit 7 ml Wasser versetzt. Es wurde mit 370 ml Ether verdünnt, zweimal mit je 120 ml Wasser und mit 60 ml gesättigter Kochsalzlösung gewaschen und die organische Phase über Natriumsulfat getrocknet. Nach dem Entfernen des Lösungsmittels wurde der Rückstand über 35 g Kieselgel mit Toluol-Essigester (19:1) chromatographiert. Man erhielt 0.5 g (35%) (RS)-1-(6-Methyl-indol-1-yl)-propan-2-ol als hellbraune Kristalle mit Smp. 65-69°.

b) Eine Lösung von 0.49 g (13.2 mmol) (RS)-1-(6-Methyl-indol-1-yl)-propan-2-ol in 10 ml Dichlormethan wurde mit 1 ml (53 mmol) Triethylamin versetzt und auf 0° gekühlt. Zu dieser Lösung wurde 0.5 ml (26.4 mmol) Methansulfonsäurechlorid zugetropft und das Reaktionsgemisch wurde eine Stunde gerührt. Man verdünnte mit Ether. Es wurde zweimal mit 1M Natriumcarbonatlösung und gesättigter Kochsalzlösung gewaschen und die wässrige Phase mit Ether nachextrahiert. Die vereinigten organischen Phasen wurden über Natriumsulfat getrocknet und das Lösungsmittel wurde entfernt. Der Rückstand wurde in 20 ml Dimethylformamid gelöst und nach der Zugabe von 0.5 g (11.4 mmol) Natriumazid 16 Stunden bei 60° gerührt. Man verdünnte mit 660 ml Ether und extrahierte zweimal mit Wasser und einmal 80 ml gesättigter Kochsalzlösung. Die Wasserphase wurde mit Ether nachextrahiert und die vereinigten organischen Phasen wurden über Natriumsulfat getrocknet. Das Lösungsmittel wurde entfernt und der Rückstand über 30 g Kieslgel mit Toluol chromatographiert. Man erhielt 0.5 g (90%) (RS)-1-(2-Azido-propyl)-5-methylindol als hellgelbes Öl.

c) Eine Lösung von 0.5 g (3 mmol) (RS)-1-(2-Azido-propyl)-6-methylindol in 20 ml Ethanol wurde über 70 mg Pd-C (5%) hydriert. Der Katalysator wurde abfiltriert und mit Ethanol gewaschen. Die Lösung wurde eingedampft und der Rückstand wurde in 100 ml Ether gelöst und mit 0.25 g (2.15 mmol) Fumarsäure versetzt und über Nacht gerührt. Die ausgefallenen Kristalle wurden abfiltriert und getrocknet. Man erhielt 0.43 g (60.5%) (RS)-2-(6-Methyl-indol-1-yl)-1-methyl-ethylamin fumarat als weisse Kristalle mit Smp. 152-153° (Zers.)

Beispiel 18

a) Eine Suspension von 0.26 g (8.75 mmol, 80%) Natriumhydrid-Dispersion in 35 ml Tetrahydrofuran wurde bei 0° mit 0.97 g (7.2 mmol) 6-Fluorindol versetzt und 1 Stunde bei dieser Temperatur gerührt. Nach der Zugabe von 1 ml (14 mmol)(RS)-Methyloxiran wurde das Reaktionsgemisch 48 Stunden bei Raumtemperatur gerührt und anschliessend mit 7 ml Wasser versetzt. Es wurde mit 180 ml Ether

verdünnt, zweimal mit je 90 ml Wasser und mit 50 ml gesättigter Kochsalzlösung gewaschen und die organische Phase über Natriumsulfat getrocknet. Nach dem Entfernen des Lösungsmittels wurde der Rückstand über 28 g Kieselgel mit Toluol-Essigester (19:1) chromatographiert. Man erhielt 1.1 g (79.3%) (RS)-1-(6-Fluor-indol-1-yl)-propan-2-ol als farbloses Öl.

b) Eine Lösung von 1.1 g (5.7 mmol) (RS)-1-(6-Fluor-indol-1-yl)-propan-2-ol in 30 ml Dichlormethan wurde mit 3.17 ml (38,7 mmol) Triethylamin versetzt und auf 0° gekühlt. Zu dieser Lösung wurde 0.88 ml (11.4 mmol) Methansulfonsäurechlorid zugetropft und das Reaktionsgemisch wurde eine Stunde gerührt. Man verdünnte mit 280 ml Ether. Es wurde zweimal mit je 70 ml 1M Natriumcarbonatlösung und 35 ml gesättigter Kochsalzlösung gewaschen und die wässrige Phase mit 140 ml Ether nachextrahiert. Die vereinigten organischen Phasen wurden über Natriumsulfat getrocknet und das Lösungsmittel wurde entfernt. Der Rückstand wurde in 20 ml Dimethylformamid gelöst und nach der Zugabe von 0.5 g (11.4 mmol) Natriumazid 7 Stunden bei 60° gerührt. Man verdünnte mit 280 ml Ether und extrahierte zweimal mit je 140 ml Wasser und einmal mit 70 ml gesättigter Kochsalzlösung. Die Wasserphase wurde mit 140 ml Ether nachextrahiert und die vereinigten organischen Phasen wurden über Natriumsulfat getrocknet. Das Lösungsmittel wurde entfernt und der Rückstand über 25 g Kieselgel mit Toluol chromatographiert. Man erhielt 1 g (80.5%) (RS)-1-(2-Azido-propyl)-6-fluorindol als gelbliches Öl.

c) Eine Lösung von 1 g (3 mmol) (RS)-1-(2-Azido-propyl)-6-fluorindol in 30 ml Ethanol wurde über 100 mg Pd-C (5%) hydriert. Der Katalysator wurde abfiltriert und mit Ethanol gewaschen. Die Lösung wurde eingedampft und der Rückstand wurde in 100 ml Ether gelöst und mit 0.5 g (4.3 mmol) Fumarsäure versetzt und über Nacht gerührt. Die ausgefallenen Kristalle wurden isoliert und getrocknet. Man erhielt 1.1 g (78%) (RS)-2-(6-Fluor-indol-1-yl)-1-methyl-ethylamin fumarat (1:1) als weisse Kristalle mit Smp. 158-159° (Zers.)

Beispiel 19

a) Zu einer Lösung von 14.5 ml (102 mmol) 2-Methylacetessigester in 100 ml Ethanol wurden bei 2-4° 40 ml 50%ige Kalilauge zugetropft. Es wurden 200 ml Eiswasser zugegeben und mit einer Diazoniumsalzlösung versetzt, die wie folgt bereitet wurde: Unter Eisbadkühlung wurden 20 ml einer 25%igen Salzsäurelösung zu einer Lösung von 10 ml (100 mmol) 3,4-Difluoranilin in 100 ml Ethanol getropft. Anschliessend wurden bei 4° 13.7 ml (102 mmol) Isopentylnitrit zugefügt. Die Emulsion, die aus der Zugabe der Diazoniumsalzlösung resuitierte, wurde auf Wasser gegossen und mit Toluol extrahiert. Die organische Phase wurden über Natriumsulfat getrocknet, filtriert und auf ein Volumen von 160 ml eingeengt. Eine Lösung von 19 g (100 mmol) p-Toluolsulfonsäure-monohydrat in 160 ml Toluol wurde, nachdem man eine Stunde am Wasserabscheider gekocht hat, zugefügt und man erhitzte die Mischung eine weitere Stunde. Nach dem Abkühlen extrahierte man mit 1N Salzsäure, 1N Natronlauge und gesättigter Kochsalzlösung. Die Wasserphasen wurden mit Toluol nachgewaschen, und die vereinigten organischen Phasen wurden über Natriumsulfat getrocknet, filtriert und eingedampft. Der Rückstand wurde über 170 g Kieselgel mit Hexan-Essigester (2:1) chromatographiert. Man erhielt 8.9 g (39%) rohen 4,5-Difluorindol-2 carbonsäure ethylester. Eine Probe wurde aus Hexan-Essigester umkristallisiert und zeigte dann einen Smp. von 149-150°.

b) Eine Lösung von 2.06 g (9.15 mmol) 5,6-Difluorindol-2-carbonsäure ethylester in 90 ml Ethanol wurde mit 45 ml 2N Natronlauge versetzt und 17 Stunden bei Raumtemperatur gerührt. Man dampfte den Alkohol ab und versetzte den Rückstand mit 50 ml 2N Salzsäure. Die ausgefallenen Kristalle wurden abfiltriert, mit Wasser gewaschen und getrocknet. Man erhielt 1.78 g (98.8%) 5,6-Difluorindol-2-carbonsäure als braune Kristalle mit Smp. 279-280°.

c) Ein Metallbad wurde auf 330° geheitzt. Man brachte 1.74 g (8.83 mmol) 5,6-Difluorindol-2-carbonsäure unter Argon ein. Der schwarze Reaktionsrückstand wurde über 60 g Kieselgel mit Hexan-Essigester (4:1) chromatographiert. Man erhielt 1.11 g (82%) 5,6-Difluorindol als hellbraune Kristalle. Eine Probe wurde sublimiert und zeigte dann einen Smp. von 89-91°.

d) Eine Suspension von 0.12 g (4.1 mmol, 80%) Natriumhydrid-Dispersion in 15 ml Tetrahydrofuran wurde bei 0° mit 0.5 g (3.2 mmol) 5,6-Difluorindol versetzt und 1 Stunde bei dieser Temperatur gerührt. Nach der Zugabe von 0.46 ml (6.5 mmol) (R)-Methyloxiran wurde das Reaktionsgemisch 60 Stunden bei Raumtemperatur gerührt und anschliessend mit Wasser versetzt. Es wurde mit Ether verdünnt, zweimal mit Wasser und mit gesättigter Kochsalzlösung gewaschen und die organische Phase über Natriumsulfat getrocknet. Nach dem Entfernen des Lösungsmittels wurde der Rückstand über 35 g Kieselgel mit Toluol-Essigester (19:1) chromatographiert. Man erhielt 0.46 g (66.5%) (R)-1-(5,6-Difluor-indol-1-yl)-propan-2-ol als gelbliche Kristalle mit Smp. 114-116°.

$[\alpha]_D^{20}$ = -44.4 (c 0.25, $CHCl_3$)

e) Eine Lösung von 0.43 g (2 mmol) (R)-1-(5,6-Difluor-indol-1-yl)-propan-2-ol in 10 ml Dichlormethan wurde mit 0.86 ml (6.2 mmol) Triethylamin versetzt und auf 0° gekühlt. Zu dieser Lösung wurde 0.32 ml (4.14 mmol) Methansulfonsäurechlorid zugetropft und das Reaktionsgemisch wurde eine Stunde gerührt. Man verdünnte mit Ether. Es wurde mit 1M Natriumcarbonatlösung und gesättigter Kochsalzlösung gewaschen und die wässrige Phase mit Ether nachextrahiert. Die vereinigten organischen Phasen wurden über Natriumsulfat getrocknet und das Lösungsmittel wurde entfernt. Der Rückstand wurde in 10 ml Dimethylformamid gelöst und nach der Zugabe von 0.26 g (4.1 mmol) Natriumazid 7 Stunden bei 60° gerührt. Man verdünnte mit Ether und extrahierte zweimal mit Wasser und einmal mit gesättigter Kochsalzlösung. Die Wasserphase wurde mit Ether nachextrahiert und die vereinigten organischen Phasen wurden über Natriumsulfat getrocknet. Das Lösungsmittel wurde entfernt und der Rückstand über 15 g Kieslgel mit Toluol chromatographiert. Man erhielt 0.4 g (83%) (S)-1-(2-Azido-propyl)-5,6-difluorindol als gelbliches Öl.
$[\alpha]_D^{20}$ = +97.2 (c 0.25, $CHCl_3$)
f) Eine Lösung von 0.37 g (1.5 mmol) (S)-1-(2-Azido-propyl)-5,6-difluorindol in 15 ml Ethanol wurde über 40 mg Pd-C (5%) hydriert. Der Katalysator wurde abfiltriert und mit Ethanol gewaschen. Die Lösung wurde eingedampft und der Rückstand wurde in 50 ml Ether gelöst und mit 0.17 g (1.46 mmol) Fumarsäure versetzt und über Nacht gerührt. Die ausgefallenen Kristalle wurden isoliert und getrocknet. Man erhielt 0.43 g (84%) (S)-2-(5,6-Difluor-indol-1-yl)-1-methyl-ethylamin fumarat (1:1) als weisse Kristalle mit Smp. 159-160° (Zers.)
$[\alpha]_D^{20}$ = +35.2 (c 0.25, $CH_3OH$)

Beispiel 20

a) Eine Suspension von 0.12 g (4.1 mmol, 80%) Natriumhydrid-Dispersion in 15 ml Tetrahydrofuran wurde bei 0° mit 0.5 g (3.2 mmol) 5,6-Difluorindol versetzt und 1 Stunde bei dieser Temperatur gerührt. Nach der Zugabe von 0.46 ml (6.5 mmol) (S)-Methyloxiran wurde das Reaktionsgemisch 60 Stunden bei Raumtemperatur gerührt und anschliessend mit Wasser versetzt. Es wurde mit Ether verdünnt, zweimal mit Wasser und mit gesättigter Kochsalzlösung gewaschen und die organische Phase über Natriumsulfat getrocknet. Nach dem Entfernen des Lösungsmittels wurde der Rückstand über 35 g Kieselgel mit Toluol-Essigester (19:1) chromatographiert. Man erhielt 0.5 g (74%) (S)-1-(5,6-Difluor-indol-1-yl)-propan-2-ol als gelbliche Kristalle mit Smp. 116-118°.
$[\alpha]_D^{20}$ = +44.8 (c 0.25, $CHCl_3$)
b) Eine Lösung von 0.48 g (2.3 mmol) (S)-1-(5,6-Difluor-indol-1-yl)-propan-2-ol in 10 ml Dichlormethan wurde mit 0.97 ml (6.9 mmol) Triethylamin versetzt und auf 0° gekühlt. Zu dieser Lösung wurden 0.36 ml (4.6 mmol) Methansulfonsäurechlorid zugetropft und das Reaktionsgemisch wurde eine Stunde gerührt. Man verdünnte mit Ether. Es wurde zweimal mit 1M Natriumcarbonatlösung und gesättigter Kochsalzlösung gewaschen und die wässrige Phase mit Ether nachextrahiert. Die vereinigten organischen Phasen wurden über Natriumsulfat getrocknet und das Lösungsmittel wurde entfernt. Der Rückstand wurde in 10 ml Dimethylformamid gelöst und nach der Zugabe von 0.29 g (4.6 mmol) Natriumazid 7 Stunden bei 60° gerührt. Man verdünnte mit Ether und extrahierte zweimal mit Wasser und einmal mit gesättigter Kochsalzlösung. Die Wasserphase wurde mit Ether nachextrahiert und die vereinigten organischen Phasen wurden über Natriumsulfat getrocknet. Das Lösungsmittel wurde entfernt und der Rückstand über 15 g Kieslgel mit Toluol chromatographiert. Man erhielt 0.48 g (89.4%) (R)-1-(2-Azido-propyl)-5,6-difluorindol als gelbliches Öl.
$[\alpha]_D^{20}$ = -98 (c 0.25, $CHCl_3$)
c) Eine Lösung von 0.45 g (1.9 mmol) (R)-1-(2-Azido-propyl)-5,6-difluorindol in 20 ml Ethanol wurde über 40 mg Pd-C (5%) hydriert. Der Katalysator wurde abfiltriert und mit Ethanol gewaschen. Die Lösung wurde eingedampft und der Rückstand wurde in 50 ml Ether gelöst und mit 0.21 g (1.8 mmol) Fumarsäure versetzt und über Nacht gerührt. Die ausgefallenen Kristalle wurden abfiltriert und getrocknet. Man erhielt 0.51 g (82%) (R)-2-(5,6-Difluor-indol-1-yl)-1-methyl-ethylamin fumarat (1:1) als weisse Kristalle mit Smp. 161-162° (Zers.)
$[\alpha]_D^{20}$ = -34.4 (c 0.25, $CH_3OH$)

Beispiel 21

a) Zu einer Lösung von 20.3 ml (143 mmol) 2-Methylacetessigester in 140 ml Ethanol wurden bei 2-4° 56 ml 50%ige Kalilauge zugetropft. Es wurden 280 ml Eiswasser zugegeben und schnell mit einer Diazoniumsalzlösung versetzt, die wie folgt bereitet wurde: Unter Eisbadkühlung wurden 28 ml einer

25%igen Salzsäurelösung zu einer Lösung von 25 g (140 mmol) 4-fluor-3-trifluormethylanilin in 140 ml Ethanol getropft. Anschliessend wurden bei 4° 19.2 ml (143 mmol) Isopentylnitrit zugefügt. Die Emulsion, die aus der Zugabe der Diazoniumsalzlösung resultierte, wurde auf Wasser gegossen und mit Toluol extrahiert. Die organische Phase wurden über Natriumsulfat getrocknet, filtriert und auf ein Volumen von 250 ml eingeengt. Eine Lösung von 26.6 g (140 mmol) p-Toluolsulfonsäure-monohydrat in 250 ml Toluol wurde, nachdem man 30 min am Wasserabscheider gekocht hat, zugefügt und man erhitzte die Mischung eine weitere Stunde. Nach dem Abkühlen extrahierte man mit 1N Salzsäure, 1N Natronlauge und gesättigter Kochsalzlösung. Die Wasserphasen wurden mit Toluol nachgewaschen, und die vereinigten organischen Phasen wurden über Natriumsulfat getrocknet, filtriert und eingedampft. Der Rückstand wurde über 200 g Kieselgel mit Toluol chromatographiert. Man erhielt 3.2 g (6.3%) rohen 5-Fluor-6-trifluormethylindol-2-carbonsäure ethylester. Eine Probe wurde mit Hexan verrührt und zeigte dann einen Smp. von 159-162°. Eine zweite Fraktion enthielt 1.4 g (3.6%) 5-Fluor-4-trifluormethylindol-2-carbonsäure ethylester als orange Kristalle mit einem Smp. von 121-124°.

b) Eine Lösung von 2.24 g (8.14 mmol) 5-Fluor-4-trifluormethylindol-2-carbonsäure ethylester in 80 ml Ethanol wurde mit 40 ml 2N Natronlauge versetzt und 1 Stunde bei Raumtemperatur gerührt. Man dampfte den Alkohol ab und stellte die Lösung mit 2N Salzsäure auf pH 1. Die ausgefallenen Kristalle wurden isoliert, mit Wasser gewaschen und getrocknet. Man erhielt 1.7 g (85 %) 5-Fluor-4-trifluormethyl-indol-2-carbonsäure als beige Kristalle mit Smp. 204-210°.

c) Eine Suspension von 0.8 g (3.2 mmol) 5-Fluor-4-trifluormethylindol-2-carbonsäure in 16 ml Diphenylether wurde 4 Stunden bei 260° gerührt und nach dem Abkühlen auf 0° mit 16 ml Tetrahydrofuran verdünnt. Man fügte 122 mg (4 mmol, 80%) Natriumhydrid-Dispersion zu und rührte eine Stunde. Anschliessend wurden 0.46 ml (6.5 mmol) (S)-Methyloxiran zugegeben und die Reaktionsmischung 60 Stunden bei Raumtemperatur gerührt. Die Mischung wurde mit Diethylether, Wasser und gesättigter Kochsalzlösung extrahiert und die organische Phase wurde über Natriumsulfat getrocknet. Das Lösungsmittel wurde entfernt und der Rückstand wurde über 40 g Kieselgel mit Hexan-Toluol (1:1) und anschliessend mit Toluol-Essigester (15:1) chromatographiert. Man erhielt 0.3 g (35.5%) R)-1-(5-Fluor-4-trifluormethyl-indol-1-yl)-propan-2-ol als hellbraune Kristalle.

$[\alpha]_D^{20}$ = +34.4 (c 0.25, $CHCl_3$)

d) Eine Lösung von 0.27 g (0.57 mmol) (R)-1-(5-Fluor-4-trifluormethyl-indol-1-yl)-propan-2-ol in 5 ml Dichlormethan wurde mit 0.44 ml (3.2 mmol) Triethylamin versetzt und auf 0° gekühlt. Zu dieser Lösung wurde 0.16 ml (2.1 mmol) Methansulfonsäurechlorid zugetropft und das Reaktionsgemisch wurde eine Stunde gerührt. Man verdünnte mit Ether. Es wurde zweimal mit 1M Natriumcarbonatlösung und gesättigter Kochsalzlösung gewaschen und die wässrige Phase mit Ether nachextrahiert. Die vereinigten organischen Phasen wurden über Natriumsulfat getrocknet und das Lösungsmittel wurde entfernt. Der Rückstand wurde in 5 ml Dimethylformamid gelöst und nach der Zugabe von 0.13 g (1.1 mmol) Natriumazid 7 Stunden bei 60° gerührt Man verdünnte mit Ether und extrahierte zweimal mit Wasser und einmal mit gesättigter Kochsalzlösung. Die Wasserphase wurde mit Ether nachextrahiert und die vereinigten organischen Phasen wurden über Natriumsulfat getrocknet. Das Lösungsmittel wurde entfernt und der Rückstand über 10 g Kieselgel mit Toluol-Hexan (1:1) chromatographiert. Man erhielt 0.26 g (87.8%) (S)-1-(2-Azido-propyl)-5-Fluor-4-trifluormethylindol als farbloses Öl.

MS: m/e (%Basispeak): 286 (M+, 12), 216 (100)

e) Eine Suspension von 26 mg Platinoxid in 4.5 ml Ethanol wurde eine halbe Stunde unter Wasserstoffatmosphäre gerührt und anschliessend mit einer Lösung von 0.26 g (0.9 mmol) (S)-1-(2-Azido-propyl)-5-fluor-4-trifluormethylindol in 4.5 ml Ethanol versetzt. Das Reaktionsgemisch wurde 1 Stunde bei Raumtemperatur gerührt. Der Katalysator wurde abfiltriert und mit Ethanol gewaschen. Die Lösung-wurde eingedampft und der Rückstand wurde in 27 ml Ether und 0.3 ml Methanol gelöst und mit 105 mg (0.9 mmol) Fumarsäure versetzt und über Nacht gerührt. Die ausgefallenen Kristalle wurden isoliert und getrocknet. Man erhielt 0.32 g (93.6%) (S)-2-(5-Fluor-4-trifluormethylindol-1-yl)-1-methyl-ethylamin fumarat (1:1) als weisse Kristalle mit Smp. 180-181° (Zers.)

$[\alpha]_D^{20}$ = +36.8 (c 0.25, $CH_3OH$)

Beispiel 22

a) Eine Lösung von 1.85 g (6.7 mmol) 5-Fluor-6-trifluormethylindol-2-carbonsäure ethylester in 60 ml Ethanol wurden mit 30 ml 2N Natronlauge versetzt und 1 Stunde bei Raumtemperatur gerührt. Man dampfte den Alkohol ab und stellte die Lösung mit 2N Salzsäure auf pH 1. Die ausgefallenen Kristalle wurden isoliert, mit Wasser gewaschen und getrocknet. Man erhielt 1.5 g (90%) 5-Fluor-6-trifluormethyl-indol-2-carbonsäure als braune Kristalle mit Smp. 178-180°.

b) Eine Suspension von 0.75 g (3 mmol) 5-Fluor-6-trifluormethylindol-2-carbonsäure in 16 ml Diphenylether wurde 4 Stunden bei 260° gerührt und nach dem Abkühlen auf 0° mit 16 ml Tetrahydrofuran verdünnt. Man fügte 113 mg (3.8 mmol, 80%) Natriumhydrid-Dispersion zu und rührte eine Stunde. Anschliessend wurden 0.43 ml (6.14 mmol) (R)-Methyloxiran zugegeben und die Reaktionsmischung 60 Stunden bei Raumtemperatur gerührt. Die Mischung wurde mit Diethylether, Wasser und gesättigter Kochsalzlösung extrahiert und die organische Phase wurde über Natriumsulfat getrocknet. Das Lösungsmittel wurde entfernt und der Rückstand wurde über 40 g Kieselgel mit Hexan-Toluol (1:1) und anschliessend mit Toluol-Essigester (15:1) chromatographiert. Man erhielt 0.15 g (18.9%) (R)-1-(5-Fluor-6-trifluormethyl-indol-1-yl)-propan-2-ol als hellbraune Kristalle.

c) Eine Lösung von 0.15 g (0.57 mmol) (R)-1-(5-Fluor-6-trifluormethyl-indol-1-yl)-propan-2-ol in 3 ml Dichlormethan wurde mit 0.24 ml (1.73 mmol) Triethylamin versetzt und auf 0° gekühlt. Zu dieser Lösung wurden 0.09 ml (1.1 mmol) Methansulfonsäurechlorid zugetropft und das Reaktionsgemisch wurde eine Stunde gerührt. Man verdünnte mit Ether. Es wurde mit 1M Natriumcarbonatlösung und gesättigter Kochsalzlösung gewaschen und die wässrige Phase mit Ether nachextrahiert. Die vereinigten organischen Phasen wurden über Natriumsulfat getrocknet und das Lösungsmittel wurde entfernt. Der Rückstand wurde in 3 ml Dimethylformamid gelöst und nach der Zugabe von 0.07 g (1.1 mmol) Natriumazid 7 Stunden bei 60° gerührt. Man verdünnte mit Ether und extrahierte zweimal mit Wasser und einmal mit gesättigter Kochsalzlösung. Die Wasserphase wurde mit Ether nachextrahiert und die vereinigten organischen Phasen wurden über Natriumsulfat getrocknet. Das Lösungsmittel wurde entfernt und der Rückstand über 8 g Kieselgel mit Toluol-Hexan (1:1) chromatographiert. Man erhielt 0.13 g (79.2%) (S)-1-(2-Azido-propyl)-5-Fluor-6-trifluormethylindol als farbloses Öl.
MS: m/e (%Basispeak): 286 (M+, 12), 216 (100)

d) Eine Suspension von 13 mg Platinoxid in 2.5 ml Ethanol wurde eine halbe Stunde unter Wasserstoffatmosphäre gerührt und anschliessend mit einer Lösung von 0.13 g (0.4 mmol) (S)-1-(2-Azido-propyl)-5-fluor-6-trifluormethylindol in 2.5 ml Ethanol versetzt. Das Reaktionsgemisch wurde 1 Stunde bei Raumtemperatur gerührt. Der Katalysator wurde abfiltriert und mit Ethanol gewaschen. Die Lösung wurde eingedampft und der Rückstand wurde in 14 ml Ether und 0.1 ml Methanol gelöst und mit 53 mg (0.45 mmol) Fumarsäure versetzt und über Nacht gerührt. Die ausgefallenen Kristalle wurden abfiltriert und getrocknet. Man erhielt 0.16 g (93.5%) (S)-2-(5-Fluor-6-trifluormethylindol-1-yl)-1-methyl-ethylamin fumarat (1:1) als weisse Kristalle mit Smp. 170-171° (Zers.)
$[\alpha]_D^{20}$ = +24 (c 0.25, $CH_3OH$)

Beispiel 23

a) Eine Suspension von 0.11 g (3.7 mmol, 80%) Natriumhydrid-Dispersion in 15 ml Tetrahydrofuran wurde bei 0° mit 0.5 g (3 mmol) 4-Chlor-5-fluorindol versetzt und 1 Stunde bei dieser Temperatur gerührt. Nach der Zugabe von 0.4 ml (6 mmol) (R)-Methyloxiran wurde das Reaktionsgemisch 48 Stunden bei Raumtemperatur gerührt und anschliessend mit Wasser versetzt. Es wurde mit Ether verdünnt, mit Wasser und mit gesättigter Kochsalzlösung gewaschen und die organische Phase über Natriumsulfat getrocknet. Nach dem Entfernen des Lösungsmittels wurde der Rückstand über 30 g Kieselgel mit Toluol-Essigester (33:1) chromatographiert. Man erhielt 0.5 g (74.5%) (R)-1-(4-Chlor-5-fluor-indol-1-yl)-propan-2-ol als gelbes Öl. MS: m/e (%Basispeak): 227, 229 (M+, 32), 182, 184 (100)

b) Eine Lösung von 0.49 g (2.1 mmol) (R)-1-(4-Chlor-5-fluorindol-1-yl)-propan-2-ol in 11 ml Dichlormethan wurde mit 0.9 ml (6.5 mmol) Triethylamin versetzt und auf 0° gekühlt. Zu dieser Lösung wurden 0.33 ml (4.3 mmol) Methansulfonsäurechlorid zugetropft und das Reaktionsgemisch wurde eine Stunde gerührt. Man verdünnte mit Ether. Es wurde mit 1M Natriumcarbonatlösung und gesättigter Kochsalzlösung gewaschen und die wässrige Phase mit Ether nachextrahiert. Die vereinigten organischen Phasen wurden über Natriumsulfat getrocknet und das Lösungsmittel wurde entfernt. Der Rückstand wurde in 8 ml Dimethylformamid gelöst und nach der Zugabe von 0.22 g (3.4 mmol) Natriumazid 7 Stunden bei 60° gerührt. Man verdünnte mit Ether und extrahierte zweimal mit Wasser und einmal mit gesättigter Kochsalzlösung. Die Wasserphase wurde mit Ether nachextrahiert und die vereinigten organischen Phasen wurden über Natriumsulfat getrocknet. Das Lösungsmittel wurde entfernt und der Rückstand über 12 g Kieselgel mit Toluol chromatographiert. Man erhielt 0.4 g (73.5%) (S)-1-(2-Azido-propyl)-4-chlor-5-fluorindol als farbloses Öl.
MS: m/e (%Basispeak): 252, 254 (M+, 34), 182, 184 (100)

c) Eine Suspension von 40 mg Platinoxid in 8 ml Ethanol wurde eine halbe Stunde unter Wasserstoffatmosphäre gerührt und anschliessend mit einer Lösung von 0.4 g (1.5 mmol) (S)-1-(2-Azido-propyl)-4-chlor-5-fluorindol in 8 ml Ethanol versetzt. Das Reaktionsgemisch wurde 1 Stunde bei Raumtemperatur

gerührt. Der Katalysator wurde abfiltriert und mit Ethanol gewaschen. Die Lösung wurde eingedampft und der Rückstand wurde in 46 ml Ether und 0.3 ml Methanol gelöst und mit 180 mg (1.55 mmol) Fumarsäure versetzt und über Nacht gerührt. Die ausgefallenen Kristalle wurden abfiltriert und getrocknet. Man erhielt 0.48 g (83.8%) (S)-2-(4-Chlor-5-fluorindol-1-yl)-1-methyl-ethylamin fumarat (1:1) als weisse Kristalle mit Smp. 183-184° (Zers.)
$[\alpha]_D^{20}$ = +32.4 (c 0.25, $CH_3OH$)

Beispiel 24

a) Eine Suspension von 0.11 g (3.7 mmol, 80%) Natriumhydrid-Dispersion in 15 ml Tetrahydrofuran wurde bei 0° mit 0.5 g (3 mmol) 4-Chlor-5-fluorindol versetzt und 1 Stunde bei dieser Temperatur gerührt. Nach der Zugabe von 0.4 ml (6 mmol) (S)-Methyloxiran wurde das Reaktionsgemisch 48 Stunden bei Raumtemperatur gerührt und anschliessend mit Wasser versetzt. Es wurde mit Ether verdünnt, mit Wasser und mit gesättigter Kochsalzlösung gewaschen und die organische Phase über Natriumsulfat getrocknet. Nach dem Entfernen des Lösungsmittels wurde der Rückstand über 30 g Kieselgel mit Toluol-Essigester (33:1) chromatographiert. Man erhielt 0.53 g (78.9%) (S)-1-(4-Chlor-5-fluor-indol-1-yl)-propan-2-ol als gelbes Öl.
MS: m/e (%Basispeak): 227, 229 (M+, 32), 182, 184 (100)
b) Eine Lösung von 0.52 g (2.3 mmol) (S)-1-(4-Chlor-5-fluorindol-1-yl)-propan-2-ol in 11 ml Dichlormethan wurde mit 0.97 ml (6.9 mmol) Triethylamin versetzt und auf 0° gekühlt. Zu dieser Lösung wurden 0.36 ml (4.6 mmol) Methansulfonsäurechlorid zugetropft und das Reaktionsgemisch wurde eine Stunde gerührt. Man verdünnte mit Ether. Es wurde mit 1M Natriumcarbonatlösung und gesättigter Kochsalzlösung gewaschen und die wässrige Phase mit Ether nachextrahiert. Die vereinigten organischen Phasen wurden über Natriumsulfat getrocknet und das Lösungsmittel wurde entfernt. Der Rückstand wurde in 8 ml Dimethylformamid gelöst und nach der Zugabe von 0.22 g (3.4 mmol) Natriumazid 7 Stunden bei 60° gerührt. Man verdünnte mit Ether und extrahierte zweimal mit Wasser und einmal mit gesättigter Kochsalzlösung. Die Wasserphase wurde mit Ether nachextrahiert und die vereinigten organischen Phasen wurden über Natriumsulfat getrocknet. Das Lösungsmittel wurde entfernt und der Rückstand über 12 g Kieselgel mit Toluol chromatographiert. Man erhielt 0.4 g (70%) (R)-1-(2-Azido-propyl)-4-chlor-5-fluorindol als farbloses Öl.
MS: m/e (%Basispeak): 252, 254 (M+, 34), 182, 184 (100)
c) Eine Suspension von 40 mg Platinoxid in 8 ml Ethanol wurde eine halbe Stunde unter Wasserstoffatmosphäre gerührt und anschliessend mit einer Lösung von 0.4 g (1.5 mmol) (S)-1-(2-Azido-propyl)-4-chlor-5-fluorindol in 8 ml Ethanol versetzt. Das Reaktionsgemisch wurde 1 Stunde bei Raumtemperatur gerührt. Der Katalysator wurde abfiltriert und mit Ethanol gewaschen. Die Lösung wurde eingedampft und der Rückstand wurde in 46 ml Ether und 0.3 ml Methanol gelöst und mit 180 mg (1.55 mmol) Fumarsäure versetzt und über Nacht gerührt. Die ausgefallenen Kristalle wurden abfiltriert und getrocknet. Man erhielt 0.48 g (83.8%) (R)-2-(4-Chlor-5-fluorindol-1-yl)-1-methyl-ethylamin fumarat (1:1) als weisse Kristalle mit Smp. 186-187° (Zers.)
$[\alpha]_D^{20}$ = -32.4 (c 0.25, $CH_3OH$)

Beispiel 25

a) Eine Suspension von 0.26 g (8.7 mmol, 80%) Natriumhydrid-Dispersion in 35 ml Tetrahydrofuran wurde bei 0° mit 0.95 g (7.25 mmol) 5-Methylindol versetzt und 1 Stunde bei dieser Temperatur gerührt. Nach der Zugabe von 1 ml (14 mmol) (RS)-Methyloxiran wurde das Reaktionsgemisch 48 Stunden bei Raumtemperatur gerührt und anschliessend mit Wasser versetzt. Es wurde mit Ether verdünnt, mit Wasser und mit gesättigter Kochsalzlösung gewaschen und die organische Phase über Natriumsulfat getrocknet. Nach dem Entfernen des Lösungsmittels wurde der Rückstand über 30 g Kieselgel mit Toluol-Essigester (19:1) chromatographiert. Man erhielt 0.86 g (62.7%) (RS)-1-(5-Methyl-indol-1-yl)-propan-2-ol als gelbes Öl.
b) Eine Lösung von 0.86 g (4.5 mmol) (RS)-1-(5-Methyl-indol-1-yl)-propan-2-ol in 20 ml Dichlormethan wurde mit 1.6 ml (11 mmol) Triethylamin versetzt und auf 0° gekühlt. Zu dieser Lösung wurde 0.6 ml (7.7 mmol) Methansulfonsäurechlorid zugetropft und das Reaktionsgemisch wurde eine Stunde gerührt. Man verdünnte mit Ether. Es wurde mit 1M Natriumcarbonatlösung und gesättigter Kochsalzlösung gewaschen und die wässrige Phase mit Ether nachextrahiert. Die vereinigten organischen Phasen wurden über Natriumsulfat getrocknet und das Lösungsmittel wurde entfernt. Der Rückstand wurde in 20 ml Dimethylformamid gelöst und nach der Zugabe von 0.6 g (9.2 mmol) Natriumazid 7 Stunden bei 60°

gerührt. Man verdünnte mit Ether und extrahierte mit Wasser und gesättigter Kochsalzlösung. Die Wasserphase wurde mit Ether nachextrahiert und die vereinigten organischen Phasen wurden über Natriumsulfat getrocknet. Das Lösungsmittel wurde entfernt und der Rückstand über 30 g Kieselgel mit Toluol chromatographiert. Man erhielt 0.68 g (71.6%) (RS)-1-(2-Azido-propyl)-5-methylindol als gelbes Öl.

MS: m/e (%Basispeak): 214 (M+,20), 144 (100)

c) Eine Lösung von 0.6 g (2.6 mmol) (RS)-1-(2-Azido-propyl)-5-methylindol in 20 ml Ethanol wurde über 50 mg Pd-C (5%) hydriert. Der Katalysator wurde abfiltriert und mit Ethanol gewaschen. Die Lösung wurde eingedampft und der Rückstand wurde in 90 ml Ether gelöst und mit 0.35 g (3 mmol) Fumarsäure versetzt und über Nacht gerührt. Die ausgefallenen Kristalle wurden isoliert und getrocknet. Man erhielt 0.83 g (87%) (RS)-2-(5-Methylindol-1-yl)-1-methyl-ethylamin fumarat (1:1) als weisse Kristalle mit Smp. 165-167° (Zers.)

Beispiel 26

a) Unter Eisbadkühlung wurden 1.35 g (58.7 mmol) Natrium in 30 ml Methanol gelöst und anschliessend wurde eine Lösung von 4.65 g ( 29.3 mmol) 3-Chlor-4-fluorbenzaldehyd und 6.75 g ( 58.6 mmol) Azidoessigsäure methylester in 10 ml Methanol innerhalb von 20 min zugegeben. Die Reaktionsmischung wurde 3 Stunden bei Raumtemperatur gerührt und dann mit 2 N HCl neutralisiert. Es wurde mit Essigester extrahiert, mit Wasser und gesättigter Kochsalzlösung gewaschen und die organische Phase über Natriumsulfat getrocknet. Das Lösungsmittel wurde entfernt und der Rückstand über 250 g Kieselgel mit n-Hexan-Toluol 2:1 chromatographiert. Man erhielt 2.5 g (33%) 3-Chlor-4-fluor-α-Azidozimtsäure methylester als gelbe Kristalle mit Smp. 72-74°.

b) Eine Mischung von 2.38 g (9.3 mmol) 3-Chlor-4-fluor-α-Azido-zimtsäure methyl-ester und 180 ml Xylol wurde 45 min unter Rückfluss erhitzt. Das Lösungsmittel wurde entfernt. Man erhielt 2.04 g (quant.) einer nahezu 1:1 Mischung von 5-Chlor-6-fluor-indol-2-carbonsäure methylester und 7-Chlor-6-fluor-indol-2-carbonsäure methylester als hellgelbe Kristalle, die ohne weitere Reinigung in die nächste Stufe eingesetzt wurde.

c) Eine Suspension von 2.04 g (9 mmol) einer nahezu 1:1 Mischung von 5-Chlor-6-fluor-indol-2-carbonsäure methylester und 7-Chlor-6-fluor-indol-2-carbonsäure methylester in 90 ml Ethanol und 45 ml 2 N Natronlauge wurde eine Stunde bei Raumtemperatur gerührt. Man dampfte den Alkohol ab und versetzte den Rückstand mit 55 ml 2 N Salzsäure. Die ausgefallenen Kristalle wurden isoliert, mit Wasser gewaschen und getrocknet. Man erhielt 1.92 g (quant.) einer nahezu 1:1 Mischung von 5-Chlor-6-fluor-indol-2-carbonsäure und 7-Chlor-6-fluor-indol-2-carbonsäure als gelbe Kristalle.

c) Eine Suspension von 1.92 g (9 mmol) einer nahezu 1:1 Mischung von 5-Chlor-6-fluor-indol-2-carbonsäure und 7-Chlor-6-fluor-indol-2-carbonsäure in 45 ml Diphenyl-ether wurde 4 Stunden bei 260° gerührt. Die Reaktionsmischung wurde über 100 g Kieselgel mit Hexan und Hexan-Toluol (3:1) chromatographiert. Man erhielt 0.6 g (39%) 5-Chlor-6-fluorindol als hellbraune Kristalle mit Smp. 88-90° und 0.22 g (14%) 7-Chlor-6-fluorindol als dunkelbraunes Öl.

d) Eine Suspension von 128 mg (4.25 mmol, 80%) Natriumhydrid-Dispersion in 8 ml Tetrahydrofuran wurde bei 0° mit 0.57 g (3.4 mmol) 5-Chlor-6-fluorindolversetzt und 1 Stunde bei dieser Temperatur gerührt. Nach der Zugabe von 0.48 ml (6.8 mmol) (R)-Methyloxiran wurde das Reaktionsgemisch 48 Stunden bei Raumtemperatur gerührt und anschliessend mit Wasser versetzt. Es wurde mit Ether verdünnt, mit Wasser und mit gesättigter Kochsalzlösung gewaschen und die organische Phase über Natriumsulfat getrocknet. Nach dem Entfernen des Lösungsmittels wurde der Rückstand über 25 g Kieselgel mit Toluol-Essigester (19:1) chromatographiert. Man erhielt 0.59 g (77%) (R)-1-(5-Chlor-6-fluor-indol-1-yl)-propan-2-ol als beige Kristalle mit Smp. 108-110°.

e) Eine Lösung von 0.57 g (2.5 mmol) (R)-1-(5-Chlor-6-fluor-indol-1-yl)-propan-2-ol in 12 ml Dichlormethan wurde mit 0.4 ml (5 mmol) Triethylamin versetzt und auf 0° gekühlt. Zu dieser Lösung wurde 0.2 ml (2.3 mmol) Methansulfonsäurechlorid zugetropft und das Reaktionsgemisch wurde eine Stunde gerührt. Man verdünnte mit Ether. Es wurde mit 1M Natriumcarbonatlösung und gesättigter Kochsalzlösung gewaschen und die wässrige Phase mit Ether nachextrahiert. Die vereinigten organischen Phasen wurden über Natriumsulfat getrocknet und das Lösungsmittel wurde entfernt. Der Rückstand wurde in 12 ml Dimethylformamid gelöst und nach der Zugabe von 0.32 g (5 mmol) Natriumazid 7 Stunden bei 60° gerührt. Man verdünnte mit Ether und extrahierte mit Wasser und gesättigter Kochsalzlösung. Die Wasserphase wurde mit Ether nachextrahiert und die vereinigten organischen Phasen wurden über Natriumsulfat getrocknet. Das Lösungsmittel wurde entfernt und der Rückstand über 15 g Kieselgel mit Toluol-n-Hexan 3:1 chromatographiert. Man erhielt 0.58 g (92%) (S)-1-(2-Azido-propyl)-5-chlor-6-fluorindol als farbloses Öl.
MS: m/e (%Basispeak): 252, 254 ($M^+$,10), 182, 184 (100)
f) Eine Suspension von 57 mg Platinoxid in 10 ml Ethanol wurde eine halbe Stunde unter Wasserstoffatmosphäre gerührt und anschliessend mit einer Lösung von 0.57 g (2.25 mmol) (S)-1-(2-Azido-propyl)-5-chlor-6-fluorindol in 10 ml Ethanol versetzt. Das Reaktionsgemisch wurde 1 Stunde bei Raumtemperatur gerührt. Der Katalysator wurde abfiltriert und mit Ethanol gewaschen. Die Lösung wurde eingedampft und der Rückstand wurde in 70 ml Ether und 2 ml Methanol gelöst und mit 262 mg (2.25 mmol) Fumarsäure versetzt und über Nacht gerührt. Die ausgefallenen Kristalle wurden isoliert und getrocknet. Man erhielt 0.63 g (82%) (S)-2-(5-Chlor-6-Fluorindol-1-yl)-1-methyl-ethylamin fumarat (1:1) als weisse Kristalle mit Smp. 159-161° (Zers.)

Beispiel 27

a) Eine Suspension von 45 mg (1.5 mmol, 80%) Natriumhydrid-Dispersion in 10 ml Tetrahydrofuran wurde bei 0° mit 0.2 g (1.2 mmol) 7-Chlor-6-fluorindolversetzt und 1 Stunde bei dieser Temperatur gerührt. Nach der Zugabe von 0.17 ml (2.4 mmol) (RS)-Methyloxiran wurde das Reaktionsgemisch 4 Tage bei Raumtemperatur gerührt und anschliessend mit Wasser versetzt. Es wurde mit Ether verdünnt, mit Wasser und mit gesättigter Kochsalzlösung gewaschen und die organische Phase über Natriumsulfat getrocknet. Nach dem Entfernen des Lösungsmittels wurde der Rückstand über 15 g Kieselgel mit Toluol-Essigester (19:1) chromatographiert. Man erhielt 0.21 g (77.8%) (RS)-1-(7-Chlor-6-fluor-indol-1-yl)-propan-2-ol als weisse Kristalle mit Smp. 85-87°.
b) Eine Lösung von 0.2 g (0.9 mmol) (RS)-1-(7-Chlor-6-fluor-indol-1-yl)-propan-2-ol in 4.5 ml Dichlormethan wurde mit 0.4 ml (2.7 mmol) Triethylamin versetzt und auf 0° gekühlt. Zu dieser Lösung wurde 0.14 ml (1.81 mmol) Methansulfonsäurechlorid zugetropft und das Reaktionsgemisch wurde eine Stunde gerührt. Man verdünnte mit Ether. Es wurde mit 1M Natriumcarbonatlösung und gesättigter Kochsalzlösung gewaschen und die wässrige Phase mit Ether nachextrahiert. Die vereinigten organischen Phasen wurden über Natriumsulfat getrocknet und das Lösungsmittel wurde entfernt. Der Rückstand wurde in 6 ml Dimethylformamid gelöst und nach der Zugabe von 0.15 g (2.3 mmol) Natriumazid 7 Stunden bei 60° gerührt Man verdünnte mit Ether und extrahierte mit Wasser und gesättigter Kochsalzlösung. Die Wasserphase wurde mit Ether nachextrahiert und die vereinigten organischen Phasen wurden über Natriumsulfat getrocknet. Das Lösungsmittel wurde entfernt und der Rückstand über 10 g Kieselgel mit Toluol-n-Hexan 1:1 chromatographiert. Man erhielt 0.17 g (80%) (RS)-1-(2-Azido-propyl)-7-chlor-6-fluorindol als farbloses Öl.
c) Eine Suspension von 17 mg Platinoxid in 4 ml Ethanol wurde eine halbe Stunde unter Wasserstoffatmosphäre gerührt und anschliessend mit einer Lösung von 0.16 g (0.65 mmol) (RS)-1-(2-Azido-propyl)-7-chlor-6-fluorindol in 4 ml Ethanol versetzt. Das Reaktionsgemisch wurde 1 Stunde bei Raumtemperatur gerührt. Der Katalysator wurde abfiltriert und mit Ethanol gewaschen. Die Lösung wurde eingedampft und der Rückstand wurde in 20 ml Ether und 0.2 ml Methanol gelöst und mit 74 mg (0.63 mmol) Fumarsäure versetzt und über Nacht gerührt. Die ausgefallenen Kristalle wurden isoliert und getrocknet. Man erhielt 0.19 g (86.5%) (RS)-2-(7-Chlor-6-fluorindol-1-yl)-1-methyl-ethylamin fumarat (1:1) als weisse Kristalle mit Smp. 187-188° (Zers.)

Beispiel 28

a) Eine Suspension von 75 mg (2.5 mmol, 80%) Natriumhydrid-Dispersion in 15 ml Tetrahydrofuran wurde bei 0° mit 0.33 g (2 mmol) 5-Chlor-2-methylindolversetzt und 1 Stunde bei dieser Temperatur gerührt. Nach der Zugabe von 0.28 ml (4 mmol) (RS)-Methyloxiran wurde das Reaktionsgemisch 48 Stunden bei Raumtemperatur gerührt und anschliessend mit Wasser versetzt. Es wurde mit Ether verdünnt, mit Wasser und mit gesättigter Kochsalzlösung gewaschen und die organische Phase über Natriumsulfat getrocknet. Nach dem Entfernen des Lösungsmittels wurde der Rückstand über 13 g Kieselgel mit Toluol-Essigester (19:1) chromatographiert. Man erhielt 0.27 g (61.7%) (RS)-1-(5-Chlor-2-methyl-indol-1-yl)-propan-2-ol als gelbes Öl.
MS: m/e (%Basispeak): 223, 225 ($M^+$,26), 178, 180 (100)
b) Eine Lösung von 0.26 g (1.1 mmol) (RS)-1-(5-Chlor-2-methyl-indol-1-yl)-propan-2-ol in 6 ml Dichlormethan wurde mit 0.5 ml (3.5 mmol) Triethylamin versetzt und auf 0° gekühlt. Zu dieser Lösung wurde 0.2 ml (2.3 mmol) Methansulfonsäurechlorid zugetropft und das Reaktionsgemisch wurde eine Stunde gerührt. Man verdünnte mit Ether. Es wurde mit 1M Natriumcarbonatlösung und gesättigter Kochsalzlösung gewaschen und die wässrige Phase mit Ether nachextrahiert. Die vereinigten organischen Phasen

wurden über Natriumsulfat getrocknet und das Lösungsmittel wurde entfernt. Der Rückstand wurde in 6 ml Dimethylformamid gelöst und nach der Zugabe von 0.15 g (2.3 mmol) Natriumazid 7 Stunden bei 60° gerührt. Man verdünnte mit Ether und extrahierte mit Wasser und gesättigter Kochsalzlösung. Die Wasserphase wurde mit Ether nachextrahiert und die vereinigten organischen Phasen wurden über Natriumsulfat getrocknet. Das Lösungsmittel wurde entfernt und der Rückstand über 10 g Kieselgel mit Toluol-n-Hexan 1:1 chromatographiert. Man erhielt 0.17 g (58.6%) (RS)-1-(2-Azido-propyl)-5-chlor-2-methylindol als farbloses Öl.

MS: m/e (%Basispeak): 248, 250 ($M^+$,16), 178. 180 (100)

c) Eine Suspension von 16 mg Platinoxid in 4 ml Ethanol wurde eine halbe Stunde unter Wasserstoffatmosphäre gerührt und anschliessend mit einer Lösung von 0.16 g (0.65 mmol) (RS)-1-(2-Azido-propyl)-5-chlor-2-methylindol in 4 ml Ethanol versetzt. Das Reaktionsgemisch wurde 1 Stunde bei Raumtemperatur gerührt. Der Katalysator wurde abfiltriert und mit Ethanol gewaschen. Die Lösung wurde eingedampft und der Rückstand wurde in 20 ml Ether und 0.2 ml Methanol gelöst und mit 74 mg (0.63 mmol) Fumarsäure versetzt und über Nacht gerührt. Die ausgefallenen Kristalle wurden isoliert und getrocknet. Man erhielt 0.19 g (86.5%) (RS)-2-(5-Chlor-2-methylindol-1-yl)-1-methyl-ethylamin fumarat (1:1) als weisse Kristalle mit Smp. 193-194° (Zers.)

Beispiel 29

a) Eine Suspension von 263 mg (8.75 mmol, 80%) Natriumhydrid-Dispersion in 35 ml Tetrahydrofuran wurde bei 0° mit 0.94 g (7 mmol) 5-Fluorindol versetzt und 1 Stunde bei dieser Temperatur gerührt. Nach der Zugabe von 1.22 ml (14 mmol) (RS)-Butylenoxid wurde das Reaktionsgemisch 96 Stunden bei Raumtemperatur gerührt und anschliessend mit Wasser versetzt. Es wurde mit Ether verdünnt, mit Wasser und mit gesättigter Kochsalzlösung gewaschen und die organische Phase über Natriumsulfat getrocknet. Nach dem Entfernen des Lösungsmittels wurde der Rückstand über 60 g Kieselgel mit Toluol chromatographiert. Man erhielt 1.2 g (83%) (RS)-5-Fluor-indol-1-yl)-butan-2-ol als gelbes Öl.

MS: m/e (%Basispeak): 207 ($M^+$,22), 148 (100)

b) Eine Lösung von 1.15 g (5.55 mmol) (RS)-5-Fluor-indol-1-yl)-butan-2-ol in 28 ml Dichlormethan wurde mit 3 ml (22 mmol) Triethylamin versetzt und auf 0° gekühlt. Zu dieser Lösung wurde 0.86 ml (11.1 mmol) Methansulfonsäurechlorid zugetropft und das Reaktionsgemisch wurde eine Stunde gerührt. Man verdünnte mit Ether. Es wurde mit 1M Natriumcarbonatlösung und gesättigter Kochsalzlösung gewaschen und die wässrige Phase mit Ether nachextrahiert. Die vereinigten organischen Phasen wurden über Natriumsulfat getrocknet und das Lösungsmittel wurde entfernt. Der Rückstand wurde in 28 ml Dimethylformamid gelöst und nach der Zugabe von 0.72 g (11.1 mmol) Natriumazid 15 Stunden bei 60° gerührt. Man verdünnte mit Ether und extrahierte mit Wasser und gesättigter Kochsalzlösung. Die Wasserphase wurde mit Ether nachextrahiert und die vereinigten organischen Phasen wurden über Natriumsulfat getrocknet. Das Lösungsmittel wurde entfernt und der Rückstand über 34 g Kieselgel mit Toluol chromatographiert. Man erhielt 1.21 g (94%) (RS)-1-(3-Azido-butyl)-5-fluorindol als gelbliches Öl.

MS: m/e (%Basispeak): 232 ($M^+$,14), 148 (100)

c) Eine Lösung von 1.18 g (5.08 mmol) (RS)-1-(3-Azido-butyl)-5-fluorindol in 25 ml Ethanol wurde über 120 mg Pd-C (5%) hydriert. Der Katalysator wurde abfiltriert und mit Ethanol gewaschen. Die Lösung wurde eingedampft und der Rückstand wurde in 150 ml Ether und 3 ml Methanol gelöst und mit 0.47 g (4.05 mmol) Fumarsäure versetzt und über Nacht gerührt. Die ausgefallenen Kristalle wurden isoliert und getrocknet. Man erhielt 1.26 g (77%) (RS)-1-Ethyl-2-(5-fluorindol-1-yl)-ethylamin fumarat (1:1) als weisse Kristalle mit Smp. 169-171° (Zers.)

Beispiel 30

a) Zu einer Lösung von 4.4 g (25.5 mmol) 2-Propylacetessigester in 26 ml Ethanol wurden bei 2-4° 10 ml 50%ige Kalilauge über einen Zeitraum von 10 min zugetropft Es wurden 50 ml Eiswasser zugegeben und schnell mit einer Diazoniumsalzlösung versetzt., die wie folgt bereitet wurde: Unter Eisbadkühlung wurden 5 ml einer 25%igen Salzsäurelösung zu einer Lösung von 2.4 ml (25 mmol) 4-Fluoranilin in 25 ml Ethanol getropft. Anschliessend wurden bei 4° innerhalb von 10 min 3.4 ml (25.5 mmol) Isopentylnitrit zugefügt. Die orange Emulsion, die aus der Zugabe der Diazoniumsalzlösung resultierte, wurde auf 100 ml Wasser gegossen und einmal mit 100 ml und zweimal mit je 50 ml Toluol extrahiert. Die vereinigten organischen Phasen wurden über Natriumsulfat getrocknet, filtriert und auf ein Volumen von 50 ml eingeengt. Eine Lösung von 7.13 g (37.5 mmol) p-Toluolsulfonsäuremonohydrat in 50 ml Toluol wurde, nachdem man 30 min am Wasserabscheider gekocht hat, zugefügt und man erhitzte die

Mischung eine weitere Stunde am Wasserabscheider. Nach dem Abkühlen extrahierte man mit 50 ml 1N Salzsäure, 50 ml 1N Natronlauge und 25 ml gesättigter Kochsalzlösung. Die Wasserphasen wurden mit 50 ml Toluol nachgewaschen, und die vereinigten organischen Phasen wurden über Natriumsulfat getrocknet, filtriert und eingedampft. Der Rückstand wurde über 105 g Kieselgel mit Toluol chromatographiert. Man erhielt 4.27 g (72.6 %) rohen 3-Ethyl-5-fluorindol-2-carbonsäure ethylester. Eine Probe wurde aus Hexan umkristallisiert und zeigte dann einen Smp. von 111-113°.

b) Eine Suspension von 1.4 g (6 mmol) 3-Ethyl-5-fluorindol-2-carbonsäure ethylester in 30 ml Ethanol wurde mit 12 ml 2N Natronlauge versetzt und 16 Stunden gerührt. Ethanol wurde abgedampft und die Reaktionslösung wurde mit 25%iger Salzsäure auf pH 1 eingestellt Der Niederschlag wurde mit Wasser gewaschen und getrocknet. Man erhielt 1.22 g (98%) rohe 3-Ethyl-5-fluorindol-2-carbonsäure, die ohne weitere Reinigung in die nächste Stufe eingesetzt wurde.

c) Eine Suspension von 1.21 g (5.8 mmol) 3-Ethyl-5-fluorindol-2-carbonsäure in 16 ml Diphenylether wurde 2 Stunden bei 260° gerührt und nach dem Abkühlen auf 0° mit 29 ml Tetrahydrofuran verdünnt. Man fügte 218 mg (7.28 mmol, 80%) Natriumhydrid-Dispersion zu und rührte eine Stunde. Anschliessend wurden 0.61 ml (8.74 mmol) (R)-Methyloxiran zugegeben und die Reaktionsmischung 90 Stunden bei Raumtemperatur gerührt. Die Mischung wurde mit Diethylether, Wasser und gesättigter Kochsalzlösung extrahiert und die organische Phase wurde über Natriumsulfat getrocknet. Das Lösungsmittel wurde entfernt und der Rückstand wurde über 120 g Kieselgel mit Hexan-Toluol (1:2) und anschliessend mit Toluol-Essigester (9:1) chromatographiert. Man erhielt 0.8 g (62 %) (R)-1-(3-Ethyl-5-fluor-indol-1-yl)-propan-2-ol als hellbraune Kristalle mit Smp. 65-67 °.

d) Eine Lösung von 0.77 g (3.48 mmol) (R)-1-(3-Ethyl-5-fluor-indol-1-yl)-propan-2-ol in 17 ml Dichlormethan wurde mit 1.9 ml (13.9 mmol) Triethylamin versetzt und auf 0° gekühlt. Zu dieser Lösung wurde 0.54 ml (6.96 mmol) Methansulfonsäurechlorid zugetropft und das Reaktionsgemisch wurde 2 Stunden gerührt. Man verdünnte mit Ether. Es wurde mit 1M Natriumcarbonatlösung und gesättigter Kochsalzlösung gewaschen und die wässrige Phase mit Ether nachextrahiert. Die vereinigten organischen Phasen wurden über Natriumsulfat getrocknet und das Lösungsmittel wurde entfernt. Der Rückstand wurde in 17 ml Dimethylformamid gelöst und nach der Zugabe von 0.45 g (6.96 mmol) Natriumazid über Nacht bei 60° gerührt. Man verdünnte mit Ether und extrahierte mit Wasser und gesättigter Kochsalzlösung. Die Wasserphase wurde mit Ether nachextrahiert und die vereinigten organischen Phasen wurden über Natriumsulfat getrocknet. Das Lösungsmittel wurde entfernt und der Rückstand wurde über 15 g Kieselgel mit Toluol-Hexan (2:1) chromatographiert. Man erhielt 0.78 g (91 %) (S)-1-(2-Azido-propyl)-3-ethyl-5-fluorindol als gelbes Öl.

MS: m/e (%Basispeak): 246 ($M^+$,20), 176 (100)

e) Eine Suspension von 76 mg Platinoxid in 15 ml Ethanol wurde eine halbe Stunde unter Wasserstoffatmosphäre gerührt und anschliessend mit einer Lösung von 0.76 g (3.09 mmol) (S)-1-(2-Azido-propyl)-3-ethyl-5-fluorindol in 15 ml Ethanol versetzt. Das Reaktionsgemisch wurde 20 Stunden bei Raumtemperatur gerührt. Der Katalysator wurde abfiltriert und mit Ethanol gewaschen. Die Lösung wurde eingedampft und der Rückstand wurde in 90 ml Ether und 1.9 ml Methanol gelöst und mit 358 mg (3 mmol) Fumarsäure versetzt und über Nacht gerührt. Die ausgefallenen Kristalle wurden isoliert und getrocknet. Man erhielt 0.91 g (88%) (S)-2-(2-Ethyl-5-fluorindol-1-yl)-1-methyl-ethylamin fumarat (1:1) als weisse Kristalle mit Smp. 164-166° (Zers.)

Beispiel 31

a) Eine Suspension von 0.16 g (6 mmol, 80%) Natriumhydrid-Dispersion in 22 ml Tetrahydrofuran wurde bei 0° mit 0.38 g (2.14 mmol) 4-Isopropyl-5-fluorindol versetzt und 1 Stunde bei dieser Temperatur gerührt. Nach der Zugabe von 0.6 ml (8.6 mmol) (R)-Methyloxiran wurde das Reaktionsgemisch 120 Stunden bei Raumtemperatur gerührt und anschliessend mit Wasser versetzt. Es wurde mit Ether verdünnt, mit Wasser und mit gesättigter Kochsalzlösung gewaschen und die organische Phase über Natriumsulfat getrocknet. Nach dem Entfernen des Lösungsmittels wurde der Rückstand über 25 g Kieselgel mit Toluol-Essigester (19:1) chromatographiert. Man erhielt 0.41 g (81 %) (R)-1-(4-Isopropyl-5-fluor-indol-1-yl)-propan-2-ol als gelbes Öl.

MS: m/e (%Basispeak): 235 ($M^+$,32), 190 (100)

b) Eine Lösung von 0.41 g (1.74 mmol) (R)-1-(4-Isopropyl-5-fluor-indol-1-yl)-propan-2-ol in 10 ml Dichlormethan wurde mit 0.74 ml (5.3 mmol) Triethylamin versetzt und auf 0° gekühlt. Zu dieser Lösung wurde 0.27 ml (3.4 mmol) Methansulfonsäurechlorid zugetropft und das Reaktionsgemisch wurde 2 Stunden gerührt. Man verdünnte mit Ether. Es wurde mit 1M Natriumcarbonatlösung und gesättigter Kochsalzlösung gewaschen und die wässrige Phase mit Ether nachextrahiert. Die vereinigten organischen Phasen wurden über Natriumsulfat getrocknet und das Lösungsmittel wurde entfernt. Der Rückstand wurde in 10 ml Dimethylformamid gelöst und nach der Zugabe von 0.22 g (3.4 mmol) Natriumazid über Nacht bei 60° gerührt. Man verdünnte mit Ether und extrahierte mit Wasser und gesättigter Kochsalzlösung. Die Wasserphase wurde mit Ether nachextrahiert und die vereinigten organischen Phasen wurden über Natriumsulfat getrocknet. Das Lösungsmittel wurde entfernt und man erhielt 0.4 g (90 %) (S)-1-(2-Azido-propyl)-4-isopropyl-5-fluorindol als gelbes Öl.
MS: m/e (%Basispeak): 260 ($M^+$,18), 190 (100)

c) Eine Lösung von 0.4 g (1.5 mmol) (S)-1-(2-Azido-propyl)-4-isopropyl-5-fluorindol in 15 ml Ethanol wurde über 40 mg Pd-C (5%) hydriert. Der Katalysator wurde abfiltriert und mit Ethanol gewaschen. Die Lösung wurde eingedampft und der Rückstand wurde in 45 ml Ether und 2 ml Methanol gelöst und mit 0.17 g (1.5 mmol) Fumarsäure versetzt und über Nacht gerührt. Die ausgefallenen Kristalle wurden isoliert und getrocknet. Man erhielt 0.43 g (80 %) (S)-2-(4-Isopropyl-5-fluorindol-1-yl)-1-methyl-ethylamin fumarat (1:1) als weisse Kristalle mit Smp. 166-167° (Zers.)

Beispiel 32

a) Eine Suspension von 0.05 g (1.6 mmol, 80%) Natriumhydrid-Dispersion in 10 ml Tetrahydrofuran wurde bei 0° mit 0.23 g (2.14 mmol) 6-Isopropyl-5-fluorindol versetzt und 1 Stunde bei dieser Temperatur gerührt. Nach der Zugabe von 0.18 ml (2.6 mmol) (R)-Methyloxiran wurde das Reaktionsgemisch 70 Stunden bei Raumtemperatur gerührt und anschliessend mit Wasser versetzt. Es wurde mit Ether verdünnt, mit Wasser und mit gesättigter Kochsalzlösung gewaschen und die organische Phase über Natriumsulfat getrocknet. Nach dem Entfernen des Lösungsmittels wurde der Rückstand über 15 g Kieselgel mit Toluol-Essigester (19:1) chromatographiert. Man erhielt 0.08 g (27 %) (R)-1-(6-Isopropyl-5-fluor-indol-1-yl)-propan-2-ol als gelbes Öl. MS: m/e (%Basispeak): 235 ($M^+$,46), 190 (100)

b) Eine Lösung von 0.08 g (0.34 mmol) (R)-1-(6-Isopropyl-5-fluor-indol-1-yl)-propan-2-ol in 1.7 ml Dichlormethan wurde mit 0.14 ml (1 mmol) Triethylamin versetzt und auf 0° gekühlt. Zu dieser Lösung wurde 0.05 ml (0.68 mmol) Methansulfonsäurechlorid zugetropft und das Reaktionsgemisch wurde 2 Stunden gerührt. Man verdünnte mit Ether. Es wurde mit 1M Natriumcarbonatlösung und gesättigter Kochsalzlösung gewaschen und die wässrige Phase mit Ether nachextrahiert. Die vereinigten organischen Phasen wurden über Natriumsulfat getrocknet und das Lösungsmittel wurde entfernt. Der Rückstand wurde in 1.7 ml Dimethylformamid gelöst und nach der Zugabe von 0.04 g (0.6 mmol) Natriumazid über Nacht bei 60° gerührt. Man verdünnte mit Ether und extrahierte mit Wasser und gesättigter Kochsalzlösung. Die Wasserphase wurde mit Ether nachextrahiert und die vereinigten organischen Phasen wurden über Natriumsulfat getrocknet. Das Lösungsmittel wurde entfernt und der Rückstand über 10 g Kieselgel mit Toluol-Hexan (1:1) chrromatographiert. Man erhielt 0.06 g (75 %) (S)-1-(2-Azido-propyl)-6-isopropyl-5-fluorindol als farbloses Öl.
MS: m/e (%Basispeak): 260 ($M^+$,22), 190 (100)

c) Eine Lösung von 0.05 g (0.2 mmol) (S)-1-(2-Azido-propyl)-6-isopropyl-5-fluorindol in 2 ml Ethanol wurde über 5 mg Pd-C (5%) hydriert. Der Katalysator wurde abfiltriert und mit Ethanol gewaschen. Die Lösung wurde eingedampft und der Rückstand wurde in 6 ml Ether gelöst und mit 0.02 g (0.17 mmol) Fumarsäure versetzt und über Nacht gerührt. Die ausgefallenen Kristalle wurden isoliert und getrocknet. Man erhielt 0.07 g (97 %) (S)-2-(6-Isopropyl-5-fluorindol-1-yl)-1-methyl-ethylamin fumarat (1:1.2) als weisse Kristalle mit Smp. 168-169° (Zers.)

Beispiel 33

a) Eine Suspension von 1.16 g (4.8 mmol) 6-Chlor-5-fluor-3-ethyl-indol-2-carbonsäure in 16 ml Diphenylether wurde 4 Stunden bei 260° gerührt und nach dem Abkühlen auf 0° mit 24 ml Tetrahydrofuran verdünnt. Man fügte 180 mg (6 mmol, 80%) Natriumhydrid-Dispersion zu und rührte eine Stunde. Anschliessend wurden 0.5 ml (7.2 mmol) (R)-Methyloxiran zugegeben und die Reaktionsmischung 112 Stunden bei Raumtemperatur gerührt. Die Mischung wurde mit Diethylether, Wasser und gesättigter Kochsalzlösung extrahiert und die organische Phase wurde über Natriumsulfat getrocknet. Das Lösungsmittel wurde entfernt und der Rückstand wurde über 120 g Kieselgel mit Hexan-Toluol (1:2) und anschliessend mit Toluol-Essigester (9:1) chromatographiert. Man erhielt 0.97 g (79 %) (R)-1-(6-Chlor-5-fluor-3-ethylindol-1-yl)-propan-2-ol als hellbraune Kristalle mit Smp. 112-114°.

b) Eine Lösung von 0.93 g (3.66 mmol) (R)-1-(6-Chlor-5-fluor-3-ethylindol-1-yl)-propan-2-ol in 18 ml Dichlormethan wurde mit 2 ml (14.6 mmol) Triethylamin versetzt und auf 0° gekühlt. Zu dieser Lösung wurde 0.57 ml (7.3 mmol) Methansulfonsäurechlorid zugetropft und das Reaktionsgemisch wurde 2

Stunden gerührt. Man verdünnte mit Ether. Es wurde mit 1M Natriumcarbonatlösung und gesättigter Kochsalzlösung gewaschen und die wässrige Phase mit Ether nachextrahiert. Die vereinigten organischen Phasen wurden über Natriumsulfat getrocknet und das Lösungsmittel wurde entfernt. Der Rückstand wurde in 18 ml Dimethylformamid gelöst und nach der Zugabe von 0.47 g (7.3 mmol) Natriumazid über Nacht bei 60° gerührt. Man verdünnte mit Ether und extrahierte mit Wasser und gesättigter Kochsalzlösung. Die Wasserphase wurde mit Ether nachextrahiert und die vereinigten organischen Phasen wurden über Natriumsulfat getrocknet. Das Lösungsmittel wurde entfernt und der Rückstand über 20 g Kieselgel mit Toluol-Hexan (2:1) chrromatographiert. Man erhielt 1 g (97 %) (S)-1-(2-Azido-propyl)-6-chlor-5-fluor-3-ethylindol als gelbes Öl.
MS: m/e (%Basispeak): 280 ($M^+$,20), 210 (100)
c) Eine Suspension von 100 mg Platinoxid in 17 ml Ethanol wurde eine halbe Stunde unter Wasserstoffatmosphäre gerührt und anschliessend mit einer Lösung von 0.97 g (3.5 mmol) (S)-1-(2-Azido-propyl)-6-chlor-5-fluor-3-ethylindol in 17 ml Ethanol versetzt. Das Reaktionsgemisch wurde 15 Stunden bei Raumtemperatur gerührt. Der Katalysator wurde abfiltriert und mit Ethanol gewaschen. Die Lösung wurde eingedampft und der Rückstand wurde in 100 ml Ether und 2 ml Methanol gelöst und mit 400 mg (3.5 mmol) Fumarsäure versetzt und über Nacht gerührt. Die ausgefallenen Kristalle wurden isoliert und getrocknet. Man erhielt 1.18 g (92 %) (S)-2-(6-Chlor-5-fluor-3-ethylindol-1-yl)-1-methyl-ethylamin fumarat (1:1) als weisse Kristalle mit Smp. 172-173° (Zers.)

Beispiel 34

a) Eine Suspension von 1.2 g (4.9 mmol) 4-Chlor-5-fluor-3-ethyl-indol-2-carbonsäure in 16 ml Diphenylether wurde 4 Stunden bei 260° gerührt und nach dem Abkühlen auf 0° mit 24 ml Tetrahydrofuran verdünnt. Man fügte 186 mg (6.2 mmol, 80%) Natriumhydrid-Dispersion zu und rührte eine Stunde. Anschliessend wurden 0.5 ml (7.2 mmol) (R)-Methyloxiran zugegeben und die Reaktionsmischung 114 Stunden bei Raumtemperatur gerührt. Die Mischung wurde mit Diethylether, Wasser und gesättigter Kochsalzlösung extrahiert und die organische Phase wurde über Natriumsulfat getrocknet. Das Lösungsmittel wurde entfernt und der Rückstand wurde über 120 g Kieselgel mit Hexan-Toluol (1:2) und anschliessend mit Toluol-Essigester (9:1) chromatographiert. Man erhielt 1.02 g (80 %) (R)-1-(4-Chlor-5-fluor-3-ethylindol-1-yl)-propan-2-ol als hellbraune Kristalle mit Smp. 87-88 °.
b) Eine Lösung von 0.98 g (3.85 mmol) (R)-1-(4-Chlor-5-fluor-3-ethylindol-1-yl)-propan-2-ol in 19 ml Dichlormethan wurde mit 2 ml (14.6 mmol) Triethylamin versetzt und auf 0° gekühlt. Zu dieser Lösung wurde 0.6 ml (7.7 mmol) Methansulfonsäurechlorid zugetropft und das Reaktionsgemisch wurde 2 Stunden gerührt. Man verdünnte mit Ether. Es wurde mit 1M Natriumcarbonatlösung und gesättigter Kochsalzlösung gewaschen und die wässrige Phase mit Ether nachextrahiert. Die vereinigten organischen Phasen wurden über Natriumsulfat getrocknet und das Lösungsmittel wurde entfernt. Der Rückstand wurde in 19 ml Dimethylformamid gelöst und nach der Zugabe von 0.5 g (7.7 mmol) Natriumazid über Nacht bei 60° gerührt. Man verdünnte mit Ether und extrahierte mit Wasser und gesättigter Kochsalzlösung. Die Wasserphase wurde mit Ether nachextrahiert und die vereinigten organischen Phasen wurden über Natriumsulfat getrocknet. Das Lösungsmittel wurde entfernt und der Rückstand über 20 g Kieselgel mit Toluol-Hexan (2:1) chromatographiert. Man erhielt 1.04 g (96 %) (S)-1-(2-Azido-propyl)-4-chlor-5-fluor-3-ethylindol als gelbes Öl.
MS: m/e (%Basispeak): 280 ($M^+$,26), 210 (100)
c) Eine Suspension von 100 mg Platinoxid in 18 ml Ethanol wurde eine halbe Stunde unter Wasserstoffatmosphäre gerührt und anschliessend mit einer Lösung von 1.02 g (3.6 mmol) (S)-1-(2-Azido-propyl)-4-chlor-5-fluor-3-ethylindol in 18 ml Ethanol versetzt. Das Reaktionsgemisch wurde 4 Stunden bei Raumtemperatur gerührt. Der Katalysator wurde abfiltriert und mit Ethanol gewaschen. Die Lösung wurde eingedampft und der Rückstand wurde in 100 ml Ether und 2 ml Methanol gelöst und mit 416 mg (3.5 mmol) Fumarsäure versetzt und über Nacht gerührt. Die ausgefallenen Kristalle wurden isoliert und getrocknet. Man erhielt 1.23 g (91 %) (S)-2-(4-Chlor-5-fluor-3-ethylindol-1-yl)-1-methyl-ethylamin fumarat (1:1) als weisse Kristalle mit Smp. 178-181° (Zers.)

Beispiel 35

a) Eine Suspension von 1.16 g (4.9 mmol) 5-fluor-3-methyl-indol-2-carbonsäure in 16 ml Diphenylether wurde 5 Stunden bei 260° gerührt und nach dem Abkühlen auf 0° mit 30 ml Tetrahydrofuran verdünnt. Man fügte 225 mg (7.5 mmol, 80%) Natriumhydrid-Dispersion zu und rührte eine Stunde. Anschliessend wurden 0.63 ml (7.2 mmol) (R)-Methyloxiran zugegeben und die Reaktionsmischung 90 Stunden bei

Raumtemperatur gerührt. Die Mischung wurde mit Diethylether, Wasser und gesättigter Kochsalzlösung extrahiert und die organische Phase wurde über Natriumsulfat getrocknet. Das Lösungsmittel wurde entfernt und der Rückstand wurde über 120 g Kieselgel mit Hexan-Toluol (1:1) und anschliessend mit Toluol-Essigester (19:1) chromatographiert. Man erhielt 0.87 g (70 %) (R)-1-(5-Fluor-3-methylindol-1-yl)-propan-2-ol als hellbraunes Öl.
MS: m/e (%Basispeak): 207 ($M^+$,22), 207 (100)
b) Eine Lösung von 0.84 g (4.05 mmol) (R)-1-(5-fluor-3-methylindol-1-yl)-propan-2-ol in 20 ml Dichlormethan wurde mit 2 ml (14.6 mmol) Triethylamin versetzt und auf 0° gekühlt. Zu dieser Lösung wurde 0.63 ml (8.1 mmol) Methansulfonsäurechlorid zugetropft und das Reaktionsgemisch wurde 2 Stunden gerührt. Man verdünnte mit Ether. Es wurde mit 1M Natriumcarbonatlösung und gesättigter Kochsalzlösung gewaschen und die wässrige Phase mit Ether nachextrahiert. Die vereinigten organischen Phasen wurden über Natriumsulfat getrocknet und das Lösungsmittel wurde entfernt. Der Rückstand wurde in 20 ml Dimethylformamid gelöst und nach der Zugabe von 0.52 g (8.1 mmol) Natriumazid über Nacht bei 60° gerührt. Man verdünnte mit Ether und extrahierte mit Wasser und gesättigter Kochsalzlösung. Die Wasserphase wurde mit Ether nachextrahiert und die vereinigten organischen Phasen wurden über Natriumsulfat getrocknet. Das Lösungsmittel wurde entfernt und der Rückstand über 90 g Kieselgel mit Toluol-Hexan (1:1) chromatographiert. Man erhielt 0.87 g (90 %) (S)-1-(2-Azido-propyl)-5-fluor-3-methylindol als farbloses Öl.
MS: m/e (%Basispeak): 280 ($M^+$,26), 210 (100)
c) Eine Lösung von 0.85 g (3.66 mmol) (S)-1-(2-Azido-propyl)-5-fluor-3-methylindol in 37 ml Ethanol wurde über 85 mg Pd-C (5%) hydriert. Der Katalysator wurde abfiltriert und mit Ethanol gewaschen. Die Lösung wurde eingedampft und der Rückstand wurde in 100 ml Ether und 5 ml Methanol gelöst und mit 0.42 g (3.6 mmol) Fumarsäure versetzt und über Nacht gerührt. Die ausgefallenen Kristalle wurden isoliert und getrocknet. Man erhielt 1.02 g (86 %) (S)-2-(5-fluor-3-methylindol-1-yl)-1-methyl-ethylamin fumarat (1:1) als weisse Kristalle mit Smp. 167-168° (Zers.)

Beispiel 36

a) Eine Suspension von 0.07 g (2.3 mmol, 80%) Natriumhydrid-Dispersion in 10 ml Tetrahydrofuran wurde bei 0° mit 0.33 g (1.82 mmol) 6-Chlor-5-fluor-3-methylindol versetzt und 1 Stunde bei dieser Temperatur gerührt. Nach der Zugabe von 0.19 ml (2.7 mmol) (R)-Methyloxiran wurde das Reaktionsgemisch 17 Stunden bei Raumtemperatur gerührt und anschliessend mit Wasser versetzt. Es wurde mit Ether verdünnt, mit Wasser und mit gesättigter Kochsalzlösung gewaschen und die organische Phase über Natriumsulfat getrocknet. Nach dem Entfernen des Lösungsmittels wurde der Rückstand über 20 g Kieselgel mit Toluol-Essigester (9:1) chromatographiert. Man erhielt 0.22 g (50 %) (R)-1-(6-Chlor-5-fluor-3-methylindol-1-yl)-propan-2-ol als gelbes Öl.
MS: m/e (%Basispeak): 241 ($M^+$,28), 196 (100)
b) Eine Lösung von 0.2 g (0.85 mmol) (R)-1-(6-Chlor-5-fluor-3-methylindol-1-yl)-propan-2-ol in 4 ml Dichlormethan wurde mit 0.47 ml (3.4 mmol) Triethylamin versetzt und auf 0° gekühlt. Zu dieser Lösung wurde 0.13 ml (1.69 mmol) Methansulfonsäurechlorid zugetropft und das Reaktionsgemisch wurde 5 Stunden gerührt. Man verdünnte mit Ether. Es wurde mit 1M Natriumcarbonatlösung und gesättigter Kochsalzlösung gewaschen und die wässrige Phase mit Ether nachextrahiert. Die vereinigten organischen Phasen wurden über Natriumsulfat getrocknet und das Lösungsmittel wurde entfernt. Der Rückstand wurde in 4 ml Dimethylformamid gelöst und nach der Zugabe von 0.1 g (1.66 mmol) Natriumazid über Nacht bei 60° gerührt. Man verdünnte mit Ether und extrahierte mit Wasser und gesättigter Kochsalzlösung. Die Wasserphase wurde mit Ether nachextrahiert und die vereinigten organischen Phasen wurden über Natriumsulfat getrocknet. Das Lösungsmittel wurde entfernt und der Rückstand über 4.5 g Kieselgel mit Toluol-Hexan (2:1) chromatographiert. Man erhielt 0.2 g (93 %) (S)-1-(2-Azido-propyl)-6-chlor-5-fluor-3-methylindol als farbloses Öl.
MS: m/e (%Basispeak): 266 ($M^+$,20), 296 (100)
c) Eine Suspension von 20 mg Platinoxid in 3.6 ml Ethanol wurde eine halbe Stunde unter Wasserstoffatmosphäre gerührt und anschliessend mit einer Lösung von 0.19 g (0.7 mmol) (S)-1-(2-Azido-propyl)-6-chlor-5-fluor-3-methylindol in 3.6 ml Ethanol versetzt. Das Reaktionsgemisch wurde 17 Stunden bei Raumtemperatur gerührt. Der Katalysator wurde abfiltriert und mit Ethanol gewaschen. Die Lösung wurde eingedampft und der Rückstand wurde in 20 ml Ether und 0.4 ml Methanol gelöst und mit 76 mg (0.65 mmol) Fumarsäure versetzt und über Nacht gerührt. Die ausgefallenen Kristalle wurden isoliert und getrocknet. Man erhielt 0.18 g (74 %) (S)-2-(6-Chlor-5-Fluor-3-methylindol-1-yl)-1-methyl-ethylamin fumarat (1:1) als weisse Kristalle mit Smp. 174-176° (Zers.)

Beispiel 37

a) Eine Suspension von 1.98 g (8.87 mmol) 5-fluor-3-methoxy-4-methyl-indol-2-carbonsäure in 16 ml Diphenylether wurde 0.5 Stunden bei 260° gerührt und nach dem Abkühlen auf 0° mit 44 ml Tetrahydrofuran verdünnt. Man fügte 0.33 g (11 mmol, 80%) Natriumhydrid-Dispersion zu und rührte eine Stunde. Anschliessend wurden 1 ml (13.3 mmol) (R)-Methyloxiran zugegeben und die Reaktionsmischung 90 Stunden bei Raumtemperatur gerührt. Die Mischung wurde mit Diethylether, Wasser und gesättigter Kochsalzlösung extrahiert und die organische Phase wurde über Natriumsulfat getrocknet. Das Lösungsmittel wurde entfernt und der Rückstand wurde über 120 g Kieselgel mit Toluol und Toluol-Essigester (9:1) chromatographiert. Man erhielt 1.69 g (80 %) (R)-1-(5-Fluor-3-methoxy-4-methylindol-1-yl)-propan-2-ol als hellbraunes Öl.
MS: m/e (%Basispeak): 237 ($M^+$,56), 192 (100)
b) Eine Lösung von 1.64 g (6.91 mmol) (R)-1-(5-fluor-3-methoxy-4-methylindol-1-yl)-propan-2-ol in 35 ml Dichlormethan wurde mit 3.85 ml (27.6 mmol) Triethylamin versetzt und auf 0° gekühlt. Zu dieser Lösung wurde 1.07 ml (13.8 mmol) Methansulfonsäure-chlorid zugetropft und das Reaktionsgemisch wurde 2 Stunden gerührt. Man verdünnte mit Ether. Es wurde mit 1M Natriumcarbonatlösung und gesättigter Kochsalzlösung gewaschen und die wässrige Phase mit Ether nachextrahiert. Die vereinigten organischen Phasen wurden über Natriumsulfat getrocknet und das Lösungsmittel wurde entfernt. Der Rückstand wurde in 30 ml Dimethylformamid gelöst und nach der Zugabe von 0.82 g (12.6 mmol) Natriumazid 3 Stunden bei 60° gerührt. Man verdünnte mit Ether und extrahierte mit Wasser und gesättigter Kochsalzlösung. Die Wasserphase wurde mit Ether nachextrahiert und die vereinigten organischen Phasen wurden über Natriumsulfat getrocknet. Das Lösungsmittel wurde entfernt und der Rückstand über 50 g Kieselgel mit Toluol chromatographiert. Man erhielt 1.82 g (77 %) (S)-1-(2-Azido-propyl)-5-fluor-3-methoxy-4-methylindol als gelbes Öl.
MS: m/e (%Basispeak): 262 ($M^+$,18), 192 (100)
c) Eine Suspension von 127 mg Platinoxid in 24 ml Ethanol wurde eine halbe Stunde unter Wasserstoffatmosphäre gerührt und anschliessend mit einer Lösung von 1.27 g (4.84 mmol) (S)-1-(2-Azido-propyl)-5-fluor-3-methoxy-4-methylindol in 3.6 ml Ethanol versetzt. Das Reaktionsgemisch wurde 15 Stunden bei Raumtemperatur gerührt. Der Katalysator wurde abfiltriert und mit Ethanol gewaschen. Die Lösung wurde eingedampft und der Rückstand wurde in 145 ml Ether und 3 ml Methanol gelöst und mit 0.56 g (4.84 mmol) Fumarsäure versetzt und über Nacht gerührt. Die ausgefallenen Kristalle wurden isoliert und getrocknet. Man erhielt 1.5 g (88 %) (S)-2-(5-Fluor-3-methoxy-4-methylindol-1-yl)-1-methyl-ethylamin fumarat (1:1) als weisse Kristalle mit Smp. 173-176° (Zers.)

Beispiel 38

a) Unter Argonbegasung wurden 1.4 g (6.8 mmol) 3-Methoxy-4-methyl-indol-2-carbonsäure 5 min auf 160° erhitzt und nach dem Abkühlen auf 0° mit 34 ml Tetrahydrofuran verdünnt. Man fügte 0.25 g (8.5 mmol, 80%) Natriumhydrid-Dispersion zu und rührte eine Stunde. Anschliessend wurden 0.72 ml (10.2 mmol) (R)-Methyloxiran zugegeben und die Reaktionsmischung 60 Stunden bei Raumtemperatur gerührt. Die Mischung wurde mit Diethylether, Wasser und gesättigter Kochsalzlösung extrahiert und die organische Phase wurde über Natriumsulfat getrocknet. Das Lösungsmittel wurde entfernt und der Rückstand wurde über 60 g Kieselgel mit Toluol-Essigester (9:1) chromatographiert. Man erhielt 0.8 g (54 %) (R)-1-(3-methoxy-4-methylindol-1-yl)-propan-2-ol als hellbraunes Öl.
b) Eine Lösung von 0.8 g (3.644 mmol) (R)-1-(3-methoxy-4-methylindol-1-yl)-propan-2-ol in 18 ml Dichlormethan wurde mit 2 ml (14.5 mmol) Triethylamin versetzt und auf 0° gekühlt. Zu dieser Lösung wurde 0.5 ml (7.28 mmol) Methansulfonsäure-chlorid zugetropft und das Reaktionsgemisch wurde 1 Stunde gerührt. Man verdünnte mit Ether. Es wurde mit 1M Natriumcarbonatlösung und gesättigter Kochsalzlösung gewaschen und die wässrige Phase mit Ether nachextrahiert. Die vereinigten organischen Phasen wurden über Natriumsulfat getrocknet und das Lösungsmittel wurde entfernt. Der Rückstand wurde in 18 ml Dimethylformamid gelöst und nach der Zugabe von 0.43 g (6.6 mmol) Natriumazid 3 Stunden bei 60° gerührt. Man verdünnte mit Ether und extrahierte mit Wasser und gesättigter Kochsalzlösung. Die Wasserphase wurde mit Ether nachextrahiert und die vereinigten organischen Phasen wurden über Natriumsulfat getrocknet. Das Lösungsmittel wurde entfernt und der Rückstand über 22 g Kieselgel mit Toluol-Hexan (1:1) chromatographiert. Man erhielt 0.46 g (57%) (S)-1-(2-Azido-propyl)-3-methoxy-4-methylindol als gelbes Öl.
MS: m/e (%Basispeak): 244 ($M^+$,16), 174 (100)

c) Eine Suspension von 45 mg Platinoxid in 9 ml Ethanol wurde eine halbe Stunde unter Wasserstoffatmosphäre gerührt und anschliessend mit einer Lösung von 0.45 g (1.84 mmol) (S)-1-(2-Azido-propyl)-3-methoxy-4-methylindol in 9 ml Ethanol versetzt. Das Reaktionsgemisch wurde 3 Stunden bei Raumtemperatur gerührt. Der Katalysator wurde abfiltriert und mit Ethanol gewaschen. Die Lösung wurde eingedampft und der Rückstand wurde in 55 ml Ether und 1 ml Methanol gelöst und mit 214 mg (1.8 mmol) Fumarsäure versetzt und über Nacht gerührt. Die ausgefallenen Kristalle wurden isoliert und getrocknet. Man erhielt 0.53 g (86 %) (S)-2-(3-Methoxy-4-methylindol-1-yl)-1-methyl-ethylamin fumarat (1:1) als hellgelbe Kristalle mit Smp. 161-162° (Zers.)

## Beispiel A

Es werden in üblicher Weise Tabletten folgender Zusammensetzung hergestellt:

| | mg/Tablette |
|---|---|
| Wirkstoff | 100 |
| Lactose pulv. | 95 |
| Maisstärke weiss | 35 |
| Polyvinylpyrrolidon | 8 |
| Na-carboxymethylstärke | 10 |
| Magnesiumstearat | 2 |
| Tablettengewicht | 250 |

## Beispiel B

Es werden in üblicher Weise Tabletten folgender Zusammensetzung hergestellt:

| | mg/Tablette |
|---|---|
| Wirkstoff | 200 |
| Lactose pulv. | 100 |
| Maisstärke weiss | 64 |
| Polyvinylpyrrolidon | 12 |
| Na-carboxymethylstärke | 20 |
| Magnesiumstearat | 4 |
| Tablettengewicht | 400 |

## Beispiel C

Es werden Kapseln folgender Zusammensetzung hergestellt:

| | mg/Kapsel |
|---|---|
| Wirkstoff | 50 |
| Lactose krist. | 60 |
| Mikrokristalline Cellulose | 34 |
| Talk | 5 |
| Magnesiumstearat | 1 |
| Kapselfüllgewicht | 150 |

Der Wirkstoff mit einer geeigneten Partikelgrösse, die Lactose kristallin und die mikrokristalline Cellulose werden homogen miteinander vermischt, gesiebt und danach Talk und Magnesiumstearat zugemischt. Die Endmischung wird in Hartgelatinekapseln geeigneter Grösse abgefüllt.

## Patentansprüche

1. Verbindungen der allgemeinen Formel

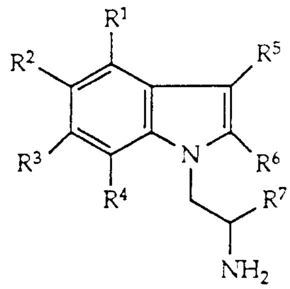

I

worin $R^1$ bis $R^4$ Wasserstoff, Halogen, nieder-Alkyl, Cycloalkyl oder Trifluormethyl, $R^5$ und $R^6$ Wasserstoff, Halogen, nieder-Alkyl, Cycloalkyl, Trifluormethyl, Hydroxy oder nieder-Alkoxy, und
$R^7$ Wasserstoff oder nieder-Alkyl bedeuten,
sowie pharmazeutisch annehmbare Säureadditionssalze der Verbindungen der Formel I.

2. Verbindungen nach Anspruch 1, worin $R^7$ nieder-Alkyl bedeutet.

3. Verbindungen nach Anspruch 2, worin $R^7$ Methyl bedeutet.

4. Verbindungen nach Anspruch 1, worin $R^7$ Wasserstoff bedeutet.

5. Verbindungen nach Anspruch 4, worin $R^5$ Wasserstoff oder Methoxy bedeutet.

6. Verbindungen nach Ansprüchen 2 oder 3, worin $R^5$ und $R^6$ Wasserstoff bedeuten.

7. Verbindungen nach einem der Ansprüche 1 bis 6, worin $R^1$ Wasserstoff oder Methyl, $R^2$ Wasserstoff oder Fluor, $R^3$ Wasserstoff oder Chlor und $R^4$ Wasserstoff bedeuten.

8. 2-(5-Fluor-indol-1-yl)-ethylamin.

9. 2-(6-Chlor-5-fluor-indol-1-yl)-ethylamin.

10. 2-(4-Methyl-3-methoxy-indol-1-yl)-ethylamin.

11. (S)-2-(6-Chlor-5-fluor-indol-1-yl)-1-methyl-ethylamin.

12. 2-(4-Chlor-5-fluor-3-methoxy-indol-1-yl)-ethylamin, 2-(5-fluor-3-methoxy-indol-1-yl)-ethylamin.

13. 2-(5-Chlor-indol-1-yl)-ethylamin, 2-(4-chlor-5-fluor-indol-1-yl)-ethylamin.

14. (RS)-2-(5-Chlor-indol-1-yl)-1-methyl-ethylamin.
(RS)-2-(5-Fluor-indol-1-yl)-1-methyl-ethylamin,
(RS)-2-(6-Chlor-5-fluor-indol-1-yl)-1-methyl-ethylamin,
(R)-2-(5-Fluor-indol-1-yl)-1-methyl-ethylamin
(S)-2-(6-Chlor-5-fluor-indol-1-yl)-1-methyl-ethylamin,
(R)-2-(6-Chlor-5-fluor-indol-1-yl)-1-methyl-ethylamin
(RS)-2-(4-Methyl-indol-1-yl)-1-methyl-ethylamin,
(RS)-2-(5-Brom-indol-1-yl)-1-methyl-ethylamin,
(RS)-2-(6-Fluor-indol-1-yl)-1-methyl-ethylamin,
(S)-2-(5,6-Difluor-indol-1-yl)-1-methyl-ethylamin,
(R)-2-(5,6-Difluor-indol-1-yl)-1-methyl-ethylamin,
(S)-2-(5-Fluor-4-Trifluormethyl-indol-1-yl)-1-methyl-ethylamin,

(S)-2-(5-Fluor-6-Trifluormethyl-indol-1-yl)-1-methyl-ethylamin,
(S)-2-(4-Chlor-5-fluor-indol-1-yl)-1-methyl-ethylamin,
(R)-2-(4-Chlor-5-fluor-indol-1-yl)-1-methyl-ethylamin.

**15.** Verbindungen der allgemeinen Formel

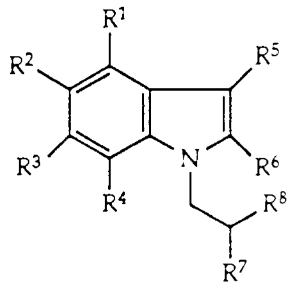

II

worin $R^1$ bis $R^7$ die in Anspruch 1 angegebenen Bedeutungen haben und $R^8$ einen in eine Aminogruppe überführbaren Rest, eine Abgangs- oder Hydroxygruppe bedeutet.

**16.** Verbindungen nach einem der Ansprüche 1 bis 14 sowie pharmazeutisch annehmbare Säureadditionssalze davon zur Anwendung als therapeutische Wirkstoffe.

**17.** Verbindungen nach einem der Ansprüche 1-14 sowie pharmazeutisch annehmbare Säureadditionssalze davon zur Anwendung als therapeutische Wirkstoffe zur Behandlung oder Verhütung von zentralnervösen Störungen, wie Depressionen, bipolaren Störungen, Angstzuständen, Schlaf- und Sexualstörungen, Psychosen, Schizophrenie, Migräne und andere mit Kopfschmerz oder Schmerz anderer Art verbundene Zustände, Persönlichkeitsstörungen oder obsessiv-compulsive Störungen, soziale Phobien oder panische Anfälle, mentale organische Störungen, mentale Störungen im Kindesalter, Aggressivität, altersbedingte Gedächtnisstörungen und Verhaltensstörungen, Sucht, Obesitas, Bulimie etc.; Schädigungen des Nervensystems durch Trauma, Schlaganfall, neurodegenerative Erkrankungen etc.; cardiovalculären Störungen, wie Bluthochdruck, Thrombose, Schlaganfall etc.; und gastrointestinalen Störungen, wie Dysfunktion der Motilität des Gastrointestinaltrakts.

**18.** Verfahren zur Herstellung von Verbindungen der allgemeinen Formel I nach einen der Ansprüche 1 bis 14 und von pharmazeutisch annehmbaren Säureadditionssalzen davon, dadurch gekennzeichnet, dass man

a) eine Verbindung der allgemeinen Formel

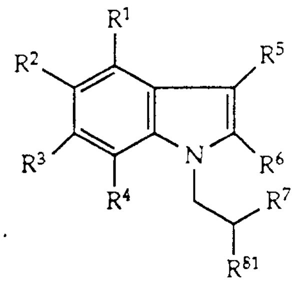

IIa

worin $R^1$ bis $R^7$ die in Anspruch 1 angegebenen Bedeutungen haben und $R^{81}$ eine in eine Aminogruppe überführbaren Rest bedeutet, in eine entsprechende Aminoverbindung überführt; oder

b) eine Verbindung der allgemeinen Formel

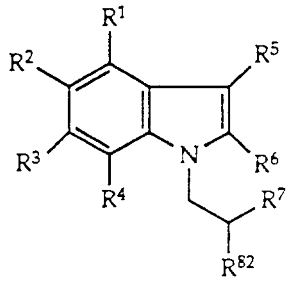

IIb

worin $R^1$ bis $R^7$ die in Anspruch 1 angegebenen Bedeutungen haben und $R^{82}$ eine Abgangsgruppe bedeutet, mit Ammoniak umsetzt; und

c) erwünschtenfalls die erhaltene Verbindung der Formel I in ein pharmazeutisch annehmbares Säureadditionssalz überführt.

**19.** Arzneimittel, enthaltend eine Verbindung nach einen der Ansprüche 1 bis 14 und ein therapeutisch inertes Trägermaterial.

**20.** Arzneimittel nach Anspruch 19 zur Behandlung oder Verhütung von zentralnervösen Störungen, wie Depressionen, bipolaren Störungen, Angstzuständen, Schlaf- und Sexualstörungen, Psychosen, Schizophrenie, Migräne und andere mit Kopfschmerz oder Schmerz anderer Art verbundenen Zustände, Persönlichkeitsstörungen oder obsessiv-compulsive Störungen, soziale Phobien oder panische Anfälle, mentale orgaische Störungen, mentale Störungen im Kindesalter, Aggressivität, altersbedingte Gedächtnisstörungen und Verhaltensstörungen, Sucht, Obesitas, Bulimie etc.; Schädigungen des Nervensystems durch Trauma, Schlaganfall, neurodegenerative Erkrankungen etc.; cardiovasculären Störungen, wie Bluthochdruck, Thrombose, Schlaganfall etc.; und gastrointestinalen Störungen, wie Dysfunktion der Motilität des Gastrointestinaltrakts.

**21.** Verwendung von Verbindungen nach einem der Ansprüche 1 bis 14 bei der Behandlung oder Verhütung von Krankheiten.

**22.** Verwendung von Verbindungen nach einem der Ansprüche 1-14 bei der Behandlung oder Verhütung von zentralnervösen Störungen, wie Depressionen, bipolaren Störungen, Angstzuständen, Schlaf- und Sexualstörungen, Psychosen, Schizophrenie, Migräne und andere mit Kopfschmerz oder Schmerz anderer Art verbundenen Zuständen, Persönlichkeitsstörungen oder obsessiv-compulsive Störungen, soziale Phobien oder panische Anfälle, mentale organische Störungen, mentale Störungen im Kindesalter, Aggressivität, altersbedingte Gedächtnisstörungen und Verhaltensstörungen, Sucht, Obesitas, Bulimie etc.; Schädigungen des Nervensystems durch Trauma, Schlaganfall, neurodegenerative Erkrankungen etc.; cardiovasculären Störungen, wie Bluthochdruck, Thrombose, Schlaganfall etc.; und gastrointestinalen Störungen, wie Dysfunktion der Motilität des Gastrointestinaltrakts bzw. zur Herstellung von entsprechenden Arzneimitteln.